(19) Europäisches Patentamt
European Patent Office
Office européen des brevets

(11) Veröffentlichungsnummer: **0 173 821**
**B1**

(12) # EUROPÄISCHE PATENTSCHRIFT

(45) Veröffentlichungstag der Patentschrift: **18.01.89**

(51) Int. Cl.[4]: **C 07 D 231/38**, A 01 N 43/56

(21) Anmeldenummer: **85108420.2**

(22) Anmeldetag: **08.07.85**

(54) **Substituierte 5-Amino-1-phenylpyrazole.**

(30) Priorität: **18.07.84 DE 3426424**

(43) Veröffentlichungstag der Anmeldung:
**12.03.86 Patentblatt 86/11**

(45) Bekanntmachung des Hinweises auf die Patenterteilung:
**18.01.89 Patentblatt 89/3**

(84) Benannte Vertragsstaaten:
**AT BE CH DE FR GB IT LI NL SE**

(56) Entgegenhaltungen:
**EP-A- 0 034 945**
**EP-A- 0 138 149**
**WO-A-83/00332**
**GB-A- 2 123 420**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Gehring, Reinhold, Dr., Dasnöckel 49, D-5600 Wuppertal 11 (DE)**
Erfinder: **Stetter, Jörg, Dr., Gellertweg 4, D-5600 Wuppertal 1 (DE)**
Erfinder: **Schallner, Otto, Dr., Noldeweg 22, D-4019 Monheim (DE)**
Erfinder: **Eue, Ludwig, Dr., Paul-Klee-Strasse 36, D-5090 Leverkusen 1 (DE)**
Erfinder: **Santel, Hans-Joachim, Dr., Gerstenkamp 19, D-5000 Köln 80 (DE)**
Erfinder: **Schmidt, Robert R. Dr., Im Waldwinkel 110, D-5060 Bergisch-Gladbach 2 (DE)**

**EP 0 173 821 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft neue substituierte 5-Amino-1-phenylpyrazole, mehrere Verfahren zu ihrer Herstellung und ihre Verwendung als Herbizide.

Es ist bereits bekannt, dass bestimmte substituierte 5-Amino-1-phenylpyrazole, wie beispielsweise das 4-Cyano-5-propionamido-1-(2,4,6-trichlorphenyl)- oder auch das entsprechende -1-(2,3,4-trichlorphenyl)-pyrazol, herbizide Eigenschaften besitzen (vgl. z.B. DE-OS 3 226 513 und GB-A 2 123 420).

Die herbizide Wirkung dieser bekannten Verbindungen gegenüber Unkräutern, ebenso wie deren Verträglichkeit gegenüber wichtigen Kulturpflanzen ist jedoch nicht immer in allen Anwendungsgebieten völlig zufriedenstellend.

Es wurden neue substituierte 5-Amino-1-phenylpyrazole der allgemeinen Formel (I) gefunden,

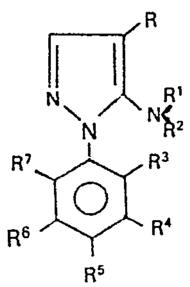

(I)

in welcher

R für Cyano, für Aminocarbonyl oder für jeweils geradkettiges oder verzweigtes Alkoxycarbonyl, Alkenyloxycarbonyl, Alkinyloxycarbonyl, Alkylaminocarbonyl, Alkenylaminocarbonyl, Alkinylaminocarbonyl, Dialkylaminocarbonyl, Dialkenylaminocarbonyl oder Dialkinylaminocarbonyl mit jeweils bis zu 5 Kohlenstoffatomen in den einzelnen aliphatischen Resten steht,

$R^1$ für jeweils gegebenenfalls einfach oder mehrfach, gleich oder verschieden substituiertes Alkyl, Alkenyl oder Alkinyl mit jeweils bis zu 8 Kohlenstoffatomen, wobei als Substituenten in Frage kommen: Halogen, Cyano, Nitro, Hydroxy, Carboxy, jeweils geradkettiges oder verzweigtes Alkoxy, oder Alkoxycarbonyl mit jeweils bis zu 6 Kohlenstoffatomen, oder gegebenenfalls durch Alkyl, Alkenyl, Alkinyl, Alkoxy oder Alkylsulfonyl substituiertes Aminocarbonyl, wobei das Stickstoffatom des Aminocarbonylrestes auch Teil eines gesättigten 3- bis 7-gliedrigen Heterocyclus mit bis zu 2 weiteren Heteroatomen, insbesondere Stickstoff, Sauerstoff oder Schwefel, sein kann; ausserdem für gegebenenfalls einfach oder mehrfach, gleich oder verschieden durch Halogen oder geradkettiges oder verzweigtes Alkyl mit 1 bis 4 Kohlenstoffatomen substituiertes Cycloalkyl mit 3 bis 7 Kohlenstoffatomen steht,

$R^2$ unabhängig von $R^1$ für die gleichen Reste wie $R^1$ steht und zusätzlich für Wasserstoff oder für einen Rest $-\underset{\underset{X}{\|}}{C}-R^8$ steht,

wobei X für Sauerstoff oder Schwefel steht und

$R^8$ für Wasserstoff, für jeweils geradkettiges oder verzweigtes Alkyl, Alkenyl, Alkinyl, Alkoxyalkyl, Alkylthioalkyl, Alkoxy, Alkylthio, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen, ausserdem für gegebenenfalls einfach oder mehrfach, gleich oder verschieden durch Halogen, niederes Alkyl oder niederes Halogenalkyl substituiertes Cycloalkyl mit 3 bis 7 Kohlenstoffatomen, sowie für jeweils gegebenenfalls einfach oder mehrfach, gleich oder verschieden substituiertes Phenyl, Phenoxy, Phenylthio oder Phenylamino steht, wobei als Phenylsubstituenten infrage kommen: Halogen, jeweils geradkettiges oder verzweigtes Alkyl, Alkoxy oder Halogenalkyl mit jeweils bis zu 4 Kohlenstoffatomen und im Fall des Halogenalkyl mit bis zu 9 gleichen oder verschiedenen Halogenatomen, und

$R^3$, $R^4$, $R^5$, $R^6$ und $R^7$ unabhängig voneinander für Wasserstoff, Fluor, Chlor, Brom, Jod, Nitro, jeweils geradkettiges oder verzweigtes Alkyl, Alkoxy, Alkylsulfonyl oder Alkoxycarbonyl mit bis zu 4 Kohlenstoffatomen in den jeweiligen Alkylteilen oder für einen Rest $-(X')_n-R^9$ stehen, wobei

X' für Sauerstoff, Schwefel, Sulfinyl oder Sulfonyl steht,

n für 0 oder 1 steht und $R^9$ für geradkettiges oder verzweigtes Halogenalkyl mit bis zu 4 Kohlenstoffatomen und bis zu 9 gleichen oder verschiedenen Halogenatomen steht, mit der Massgabe, dass mindestens einer der Reste $R^3$, $R^4$, $R^5$, $R^6$ oder $R^7$ für einen Rest $-(X')_n-R^9$ steht, wobei jedoch nicht gleichzeitig R für Cyano und $R^5$ für Trifluormethyl stehen.

Weiterhin wurde gefunden, dass man die neuen substituierten 5-Amino-1-phenylpyrazole der allgemeinen Formel (I) erhält, wenn man

(a) 5-Amino-pyrazole der Formel (II)

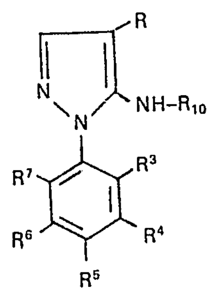

(II)

in welcher

R, $R^3$, $R^4$, $R^5$, $R^6$ und $R^7$ die oben angegebene Bedeutung haben und

$R^{10}$ für Wasserstoff oder für einen Rest $-\underset{\underset{X}{\|}}{C}-R^8$

steht,

wobei X und $R^8$ die oben angegebene Bedeutung haben, mit Alkylierungsmitteln der Formel (III)

$$R^1-A \qquad (III)$$

in welcher

$R^1$ die oben angegebene Bedeutung hat und

A für eine elektronenanziehende Abgangsgruppe steht, gegebenenfalls in Gegenwart eines Verdünnungsmittels und gegebenenfalls in Gegenwart eines Säurebindemittels sowie gegebenenfalls in Gegenwart eines Katalysators umsetzt, oder wenn man

(b) 5-Acylaminopyrazole der Formel (Ia)

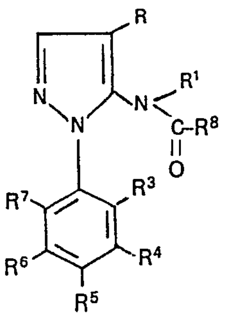

(Ia)

in welcher

R, $R^1$, $R^3$, $R^4$, $R^5$, $R^6$, $R^7$ und $R^8$ die oben angegebene Bedeutung haben, in üblicher Weise mit Säuren und Basen, gegebenenfalls in Gegenwart eines Verdünnungsmittels, deacyliert oder wenn man

(c) Azomethine der Formel (IV)

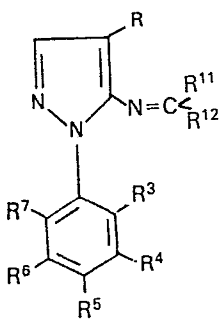

(IV)

in welcher

R, $R^3$, $R^4$, $R^5$, $R^6$, und $R^7$ die oben angegebene Bedeutung haben,

$R^{11}$ für jeweils geradkettiges oder verzweigtes, gegebenenfalls einfach oder mehrfach, gleich oder verschieden substituiertes Alkyl, Alkenyl oder Alkinyl mit jeweils bis zu 7 Kohlenstoffatomen steht, wobei als Substituenten in Frage kommen: Halogen, Cyano, Hydroxy, Carboxy sowie jeweils geradkettiges oder verzweigtes Alkoxy oder Alkoxycarbonyl mit jeweils bis zu 6 Kohlenstoffatomen, und

$R^{12}$ unabhängig von $R^{11}$ für die gleichen Reste wie dieses und ausserdem für Wasserstoff steht, mit einem komplexen Hydrid, gegebenenfalls in Gegenwart eines Verdünnungsmittels, reduziert.

Schliesslich wurde gefunden, dass die neuen substituierten 5-Amino-1-phenylpyrazole der Formel (I) herbizide Eigenschaften, insbesondere auch selektiv-herbizide Eigenschaften besitzen.

Überraschenderweise besitzen die neuen substituierten 5-Amino-1-phenylpyrazole der Formel (I) eine bessere herbizide Wirksamkeit gegenüber Schadpflanzen, bei gleichzeitiger besserer Verträglichkeit gegenüber wichtigen Nutzpflanzen, als beispielsweise die aus dem Stand der Technik bekannten Verbindungen 4-Cyano-5-propionamido-1- (2,4,6-trichlorphenyl)- bzw. -(2,3,4-trichlorphenyl)-pyrazol, welche chemisch und wirkungsmässig naheligende Verbindungen sind.

Die neuen substituierten 5-Amino-1-phenylpyrazole sind durch die Formel (I) allgemein definiert. Bevorzugt sind Verbindungen der Formel (I), bei welchen

R für Cyano, Methoxycarbonyl, Ethoxycarbonyl, Allyloxycarbonyl, Propargyloxycarbonyl, Aminocarbonyl, Methylaminocarbonyl, Dimethylaminocarbonyl, Ethylaminocarbonyl, Diethylaminocarbonyl, Diallylaminocarbonyl oder Dipropargylaminocarbonyl steht,

$R^1$ für jeweils gegebenenfalls ein- bis dreifach, gleich oder verschieden substituiertes Methyl, Ethyl, n- oder i-Propyl, n-, i-, s- oder t-Butyl, Allyl, Butenyl, Propargyl oder Butinyl steht, wobei als Substituenten in Frage kommen: Fluor, Chlor, Brom Jod, Cyano, Nitro, Hydroxy, Carboxy, Methoxy, Ethoxy, n- oder i-Propoxy, Methoxycarbonyl, Ethoxycarbonyl, n- oder i-Propoxycarbonyl, n-, i-, s- oder t-Butoxycarbonyl, Aminocarbonyl, N-Methylaminocarbonyl, N-Ethylaminocarbonyl, N,N-Dimethylaminocarbonyl, N,N-Diethylaminocarbonyl, N,N-Diallylaminocarbonyl, N-Methyl-N-ethylaminocarbonyl, N-Methyl-N-propylaminocarbonyl, N-Methyl-N-allylaminocarbonyl, N-Methyl-N-propargylaminocarbonyl, N-Methyl-N-methoxyaminocarbonyl, N-Methylsulfonylaminocarbonyl, N-Ethylsulfonylaminocarbonyl, Pyrrolidin-1-ylcarbonyl, Piperidin-1-ylcarbonyl, Morpholin-4-ylcarbonyl oder Perhydroazepin-1-ylcarbonyl; ausserdem für jeweils gegebenenfalls ein- bis fünffach gleich oder verschieden durch Chlor, Methyl, oder Ethyl substituiertes Cyclopropyl, Cyclopentyl, Cyclohexyl oder Cycloheptyl steht,

$R^2$ unabhängig von $R^1$ für die gleichen Reste wie $R^1$ steht und zusätzlich für Wasserstoff oder für einen Rest $-\underset{\underset{X}{\|}}{C}-R^8$ steht, wobei

X für Sauerstoff oder Schwefel steht und

$R^8$ für Wasserstoff, Methyl, Ethyl, n- und i-Propyl, Allyl, Propargyl, Butenyl, Methoxymethyl, Ethoxymethyl, Methoxyethyl, Ethoxyethyl, Methylthiomethyl, Methoxy, Ethoxy, Methylthio, Ethylthio, Methylamino, Ethylamino, Dimethylamino, Trifluormethyl, Trichlorethyl, Dichlorfluorethyl, Difluorchlorethyl, Chlormethyl, Jodmethyl, Brommethyl, Dichlormethyl, 1-Chlorethyl, 2-Chlorethyl, 2-Bromethyl, Heptafluor-n-propyl, für jeweils gegebenenfalls ein- bis vierfach, gleich oder verschieden durch Fluor, Chlor, Brom, Methyl- oder Trifluormethyl substituiertes Cyclopropyl, Cyclopentyl oder Cyclohexyl, sowie für jeweils gegebenenfalls ein- bis dreifach, gleich oder verschieden durch Methyl, Methoxy, Chlor oder Trifluormethyl substituiertes Phenyl, Phenoxy, Phenylthio oder Phenylamino steht, und

$R^3$, $R^4$, $R^5$, $R^6$ und $R^7$ unabhängig voneinander für Wasserstoff, Fluor, Chlor, Brom, Cyano, Nitro, Methyl, Ethyl, n- und i-Propyl, Methoxy, Ethoxy, Methylsulfonyl, Methoxycarbonyl, Ethoxycarbonyl oder für einen Rest $-(X')_n-R^9$ stehen,

wobei X' für Sauerstoff, Schwefel, Sulfinyl oder Sulfonyl steht,

n für 0 oder 1 steht und

$R^9$ für Trifluormethyl, Trichlormethyl, Dichlorfluormethyl, Difluorchlormethyl, Dichlormethyl, Chlormethyl, Difluormethyl, Pentafluorethyl, Tetrafluorethyl, Trifluorchlorethyl, Trifluorethyl, Difluordichlorethyl, Trifluordichlorethyl oder Pentachlorethyl stehen, mit der Massgabe, dass mindestens einer der Reste $R^3$, $R^4$, $R^5$, $R^6$ oder $R^7$ für einen Rest $-(X')_n-R^9$ steht, wobei jedoch nicht gleichzeitig R für Cyano und $R^5$ für Trifluormethyl stehen.

Im einzelnen seien ausser den bei den Herstellungsbeispielen genannten Verbindungen, die folgenden Verbindungen der allgemeinen Formel (I) genannt:

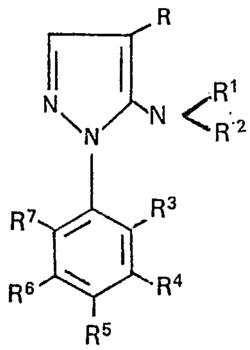

(I)

Tabelle 1:

| R | $R^1$ | $R^2$ | $R^3$ | $R^4$ | $R^5$ | $R^6$ | $R^7$ |
|---|---|---|---|---|---|---|---|
| CN | $CH_3$ | H | F | H | $OCF_3$ | H | H |
| CN | $CH_3$ | H | F | H | $OCF_3$ | H | F |
| CN | $CH_3$ | H | F | F | $OCF_3$ | F | F |
| CN | $CH_3$ | H | Cl | H | $OCF_3$ | H | H |
| CN | $CH_3$ | H | Cl | H | $OCF_3$ | H | Cl |
| CN | $CH_3$ | H | Cl | Cl | $OCF_3$ | H | H |
| CN | $CH_3$ | H | Cl | H | $OCF_3$ | H | F |
| CN | $CH_3$ | H | Br | H | $OCF_3$ | H | H |
| CN | $CH_3$ | H | Br | H | $OCF_3$ | H | Br |
| CN | $C_2H_5$ | F | H | H | $OCF_3$ | H | H |
| CN | $C_2H_5$ | H | F | H | $OCF_3$ | H | F |
| CN | $C_2H_5$ | H | F | F | $OCF_3$ | F | F |
| CN | $C_2H_5$ | $CH_3$ | Cl | H | $OCF_3$ | H | H |
| CN | $C_2H_5$ | H | Cl | Cl | $OCF_3$ | H | H |
| CN | $C_2H_5$ | H | Cl | H | $OCF_3$ | H | F |
| CN | $C_2H_5$ | H | Br | H | $OCF_3$ | H | H |
| CN | $C_2H_5$ | H | Br | H | $OCF_3$ | H | Br |
| CN | $C_2H_5$ | $CH_3$ | Cl | H | $OCF_3$ | H | Cl |
| CN | 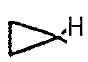 | H | F | H | $OCF_3$ | H | H |
| CN | 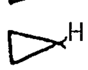 | $CH_3$ | F | H | $OCF_3$ | H | F |
| CN | 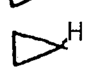 | H | F | F | $OCF_3$ | F | F |
| CN | 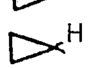 | H | Cl | H | $OCF_3$ | H | H |
| CN | 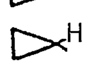 | H | Cl | H | $OCF_3$ | H | Cl |
| CN | 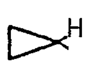 | H | Cl | Cl | $OCF_3$ | H | H |
| CN | 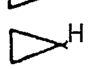 | H | Cl | H | $OCF_3$ | H | F |
| CN | 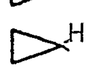 | H | Br | H | $OCF_3$ | H | H |
| CN | 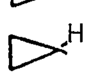 | H | Br | H | $OCF_3$ | H | Br |
| CN | $CH_3$ | H | Cl | H | $SCH_2CF_3$ | H | H |

Tabelle 1 (Fortsetzung):

| R | $R^1$ | $R^2$ | $R^3$ | $R^4$ | $R^5$ | $R^6$ | $R^7$ |
|---|---|---|---|---|---|---|---|
| CN | $CH_3$ | H | Cl | H | $SCH_2CF_3$ | H | Cl |
| CN | $CH_3$ | H | Br | H | $SCH_2CF_3$ | H | H |
| CN | $CH_3$ | H | Br | H | $SCH_2CF_3$ | H | Br |
| CN | $CH_3$ | H | Cl | Cl | $SCH_2CF_3$ | H | H |
| CN | $C_2H_5$ | H | Cl | H | $SCH_2CF_3$ | H | H |
| CN | $C_2H_5$ | H | Cl | H | $SCH_2CF_3$ | H | Cl |
| CN | $C_2H_5$ | H | Br | H | $SCH_2CF_3$ | H | H |
| CN | $C_2H_5$ | H | Br | H | $SCH_2CF_3$ | H | Br |
| CN | $C_2H_5$ | H | Cl | Cl | $SCH_2CF_3$ | H | H |
| CN | 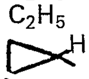 | H | Cl | H | $SCH_2CF_3$ | H | H |
| CN | 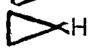 | H | Cl | H | $SCH_2CF_3$ | H | Cl |
| CN | 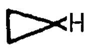 | H | Br | H | $SCH_2CF_3$ | H | H |
| CN | 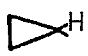 | H | Br | H | $SCH_2CF_3$ | H | Br |
| CN | 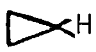 | H | Cl | Cl | $SCH_2CF_3$ | H | H |
| CN | $CH_3OCH_2$ | H | Cl | H | $SCH_2CF_3$ | H | H |
| CN | $CH_3OCH_2$ | H | Cl | H | $SCH_2CF_3$ | H | Cl |
| CN | $CH_3OCH_2$ | H | Br | H | $SCH_2CF_3$ | H | H |
| CN | $CH_3OCH_2$ | H | Br | H | $SCH_2CF_3$ | H | Br |
| CN | $CH_3OCH_2$ | H | Cl | Cl | $SCH_2CF_3$ | H | H |
| CN | $CH_3$ | H | Cl | H | $OCH_2CF_3$ | H | H |
| CN | $CH_3$ | H | Cl | H | $OCH_2CF_3$ | H | Cl |
| CN | $CH_3$ | H | Br | H | $OCH_2CF_3$ | H | H |
| CN | $CH_3$ | H | Br | H | $OCH_2CF_3$ | H | Br |
| CN | $C_2H_5$ | H | Cl | H | $OCH_2CF_3$ | H | H |
| CN | $C_2H_5$ | H | Cl | H | $OCH_2CF_3$ | H | Cl |
| CN | $C_2H_5$ | H | Br | H | $OCH_2CF_3$ | H | H |
| CN | $C_2H_5$ | H | Br | H | $OCH_2OCF_3$ | H | Br |
| CN | 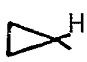 | H | Cl | H | $OCH_2CF_3$ | H | H |
| CN | 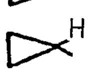 | H | Cl | H | $OCH_2CF_3$ | H | Cl |
| CN | 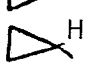 | H | Br | H | $OCH_2CF_3$ | H | H |
| CN | $C_2H_5$ | H | Cl | H | $SCF_3$ | H | H |
| CN | $C_2H_5$ | H | Cl | H | $SCF_3$ | H | Cl |
| CN | $C_2H_5$ | H | Cl | H | $SCF_3$ | H | F |
| CN | $C_2H_5$ | H | Br | H | $SCF_3$ | H | H |
| CN | 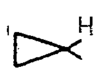 | H | F | H | $SCF_3$ | H | H |
| CN | 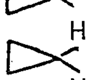 | H | F | H | $SCF_3$ | H | F |
| CN | 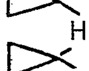 | H | F | F | $SCF_3$ | F | F |
| CN | 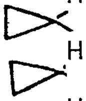 | H | Cl | H | $SCF_3$ | H | H |
| CN | 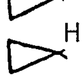 | H | Cl | H | $SCF_3$ | H | Cl |
| CN | 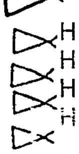 | H | Cl | Cl | $SCF_3$ | H | H |
| CN | | H | Cl | H | $SCF_3$ | H | F |
| CN | | H | Br | H | $SCF_3$ | H | H |
| CN | | H | Br | H | $SCF_3$ | H | Br |

Tabelle 1 (Fortsetzung):

| R | $R^1$ | $R^2$ | $R^3$ | $R^4$ | $R^5$ | $R^6$ | $R^7$ |
|---|---|---|---|---|---|---|---|
| CN | $CH_3$ | H | $CF_3$ | H | $SO_2CH_3$ | H | H |
| CN | $CH_3$ | H | $CF_3$ | H | $SO_2CH_3$ | H | H |
| CN | $CH_3$ | H | $CF_3$ | H | $SCF_3$ | H | H |
| CN | $CH_3$ | H | $OCF_3$ | H | $OCF_3$ | H | H |
| CN | $C_2H_5$ | H | $CF_3$ | H | $SO_2CH_3$ | H | H |
| CN | $C_2H_5$ | H | $CF_3$ | H | $SO_2CH_3$ | H | H |
| CN | $C_2H_5$ | H | $CF_3$ | H | $SCF_3$ | H | H |
| CN | $C_2H_5$ | H | $OCF_3$ | H | $OCF_3$ | H | H |
| CN | 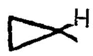 | H | $CF_3$ | H | $SO_2CH_3$ | H | H |
| CN | 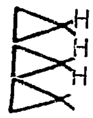 | $CH_3$ | $CF_3$ | H | $SO_2CH_3$ | H | H |
| CN | | $C_2H_5$ | $CF_3$ | H | $SCF_3$ | H | H |
| CN | | $C_3H_7$ | $OCF_3$ | H | $OCF_3$ | H | H |
| CN | $CH_3$ | $CH_3$ | Cl | H | $SCHF_2$ | H | H |
| CN | $CH_3$ | $C_2H_5$ | Cl | H | $SCH_2F$ | H | Cl |
| CN | $CH_3$ | $C_3H_7$ | Br | H | $SCHF_2$ | H | H |
| CN | $CH_3$ | H | Br | H | $SCHF_2$ | H | Br |
| CN | $C_2H_5$ | $CH_3$ | Cl | H | $SCH_2F$ | H | H |
| CN | $C_2H_5$ | $C_2H_5$ | Cl | H | $SCHF_2$ | H | Cl |
| CN | $C_2H_5$ | $C_2H_5$ | Br | H | $SCHF_2$ | H | H |
| CN | $C_2H_5$ | $C_3H_7$ | Br | H | $SCHF_2$ | H | Br |
| CN | 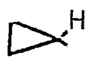 | $C_3H_7$ | Cl | H | $SCHF_2$ | H | H |
| CN | 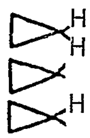 | H | Cl | H | $SCHF_2$ | H | Cl |
| CN | | $CH_3$ | Br | H | $SCHF_2$ | H | H |
| CN | | $C_3H_7$ | Br | H | $SCHF_2$ | H | Br |
| CN | $CH_3$ | H | Cl | H | $SCF_2CHF_2$ | H | H |
| CN | $CH_3$ | H | Cl | H | $SCF_2CHF_2$ | H | Cl |
| CN | $CH_3$ | H | Br | H | $SCF_2CHF_2$ | H | H |
| CN | $CH_3$ | $CH_3$ | Br | H | $SCF_2CHF_2$ | H | Br |
| CN | $C_2H_5$ | $CH_3$ | Cl | H | $SCF_2CHF_2$ | H | H |
| CN | $C_2H_5$ | $CH_3$ | Cl | H | $SCF_2CHF_2$ | H | Cl |
| CN | $C_2H_5$ | $C_2H_5$ | Br | H | $SCF_2CHF_2$ | H | H |
| CN | $C_2H_5$ | $C_2H_5$ | Br | H | $SCF_2CHF_2$ | H | Br |
| CN | 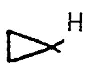 | $C_2H_5$ | Cl | H | $SCF_2CHF_2$ | H | H |
| CN | 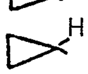 | $C_3H_7$ | Cl | H | $SCF_2CHF_2$ | H | Cl |
| CN | 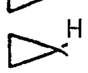 | $C_3H_7$ | Br | H | $SCF_2CHF_2$ | H | H |
| CN | 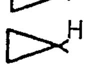 | $C_3H_7$ | Br | H | $SCF_2CHF_2$ | H | Br |
| CN | $CH_3$ | H | Cl | H | $SCF_2CHFCl$ | H | H |
| CN | $CH_3$ | H | Cl | H | $SCF_2CHFCl$ | H | Cl |
| CN | $CH_3$ | H | Br | H | $SCF_2CHFCl$ | H | H |
| CN | $CH_3$ | H | Br | H | $SCF_2CHFCl$ | H | Br |
| CN | $C_2H_5$ | H | Cl | H | $SCF_2CHFCl$ | H | H |
| CN | $C_2H_5$ | H | Cl | H | $SCF_2CHFCl$ | H | Cl |
| CN | $C_2H_5$ | H | Br | H | $SCF_2CHFCl$ | H | H |
| CN | $C_2H_5$ | H | Br | H | $SCF_2CHFCl$ | H | Br |
| CN | 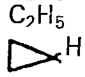 | H | Cl | H | $SCF_2CHFCl$ | H | H |
| CN | 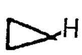 | H | Cl | H | $SCF_2CHFCl$ | H | Cl |

Tabelle 1 (Fortsetzung):

| R | $R^1$ | $R^2$ | $R^3$ | $R^4$ | $R^5$ | $R^6$ | $R^7$ |
|---|---|---|---|---|---|---|---|
| CN | 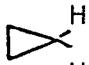 | H | Br | H | $SCF_2CHFCl$ | H | H |
| CN | 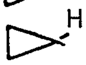 | H | Br | H | $SCF_2CHFCl$ | H | Br |
| CN | $ClCH_2$ | $CH_3$ | Cl | H | $SCF_3$ | H | H |
| CN | $ClCH_2$ | $CH_3$ | Cl | H | $OCF_3$ | H | Cl |
| CN | $ClCH_2$ | $C_2H_5$ | Br | H | $OCF_3$ | H | H |
| CN | $ClCH_2$ | H | Br | H | $OCF_3$ | H | Br |
| CN | $CH_3OCH_2$ | $C_3H_7$ | Cl | H | $OCF_3$ | H | H |
| CN | $CH_3OCH_2$ | H | Cl | H | $OCF_3$ | H | Cl |
| CN | $ClCH_2$ | H | Cl | H | $SCF_3$ | H | H |
| CN | $ClCH_2$ | H | Cl | H | $SCF_3$ | H | Cl |
| CN | $ClCH_2$ | H | Br | H | $SCF_3$ | H | H |
| CN | $CH_3$ | $CH_3$ | Cl | H | $S(O)CF_3$ | H | H |
| CN | $CH_3$ | $CH_3$ | Cl | H | $S(O)CF_3$ | H | Cl |
| CN | $CH_3$ | $C_3H_7$ | Br | H | $S(O)CF_3$ | H | Br |
| CN | $CH_3$ | $C_2H_5$ | Br | H | $S(O)CF_3$ | H | H |
| CN | $CH_3$ | $C_3H_7$ | $CF_3$ | H | $S(O)CF_3$ | H | H |
| CN | $CH_3$ | H | $CF_3$ | H | $S(O)CF_3$ | H | Cl |
| CN | $C_2H_5$ | H | Cl | H | $S(O)CF_3$ | H | H |
| CN | $C_2H_5$ | $CH_3$ | Cl | H | $S(O)CF_3$ | H | Cl |
| CN | $C_2H_5$ | $CH_3$ | Br | H | $S(O)CF_3$ | H | Br |
| CN | $C_2H_5$ | H | Br | H | $S(O)CF_3$ | H | H |
| CN | $C_2H_5$ | H | $CF_3$ | H | $S(O)CF_3$ | H | H |
| CN | $C_2H_5$ | H | $CF_3$ | H | $S(O)CF_3$ | H | Cl |
| CN | 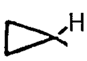 | H | Cl | H | $S(O)CF_3$ | H | H |
| CN | 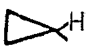 | H | Cl | H | $S(O)CF_3$ | H | Cl |
| CN | 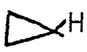 | H | Br | H | $S(O)CF_3$ | H | Br |
| CN | 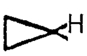 | H | Br | H | $S(O)CF_3$ | H | H |
| CN | 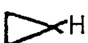 | H | $CF_3$ | H | $S(O)CF_3$ | H | H |
| CN | 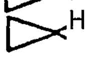 | H | $CF_3$ | H | $S(O)CF_3$ | H | Cl |
| CN | $CH_3$ | H | Cl | H | $OCF_2CHFCl$ | H | H |
| CN | $CH_3$ | H | Cl | H | $OCF_2CHFCl$ | H | Cl |
| CN | $CH_3$ | $CH_3$ | Br | H | $OCF_2CHFCl$ | H | H |
| CN | $CH_3$ | $C_2H_5$ | Br | H | $OCF_2CHFCl$ | H | Br |
| CN | $C_2H_5$ | $C_3H_7$ | Cl | H | $OCF_2CHFCl$ | H | H |
| CN | $C_2H_5$ | $C_2H_5$ | Cl | H | $OCF_2CHFCl$ | H | Cl |
| CN | $C_2H_5$ | $CH_3$ | Br | H | $OCF_2CHFCl$ | H | H |
| CN | $C_2H_5$ | H | Br | H | $OCF_2CHFCl$ | H | Br |
| CN | 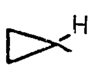 | H | Cl | H | $OCF_2CHFCl$ | H | H |
| CN | 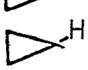 | H | Cl | H | $OCF_2CHFCl$ | H | Cl |
| CN | 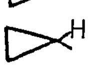 | H | Br | H | $OCF_2CHFCl$ | H | H |
| CN | 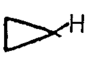 | H | Br | H | $OCF_2CHFCl$ | H | Br |
| CN | $CH_3$ | $CH_3$ | Cl | H | $OCF_2CHCl_2$ | H | H |
| CN | $CH_3$ | $C_2H_5$ | Cl | H | $OCF_2CHCl_2$ | H | Cl |
| CN | $CH_3$ | $C_3H_7$ | Br | H | $OCF_2CHCl_2$ | H | H |
| CN | $CH_3$ | H | Br | H | $OCF_2CHCl_2$ | H | Br |
| CN | $C_2H_5$ | H | Cl | H | $OCF_2CHCl_2$ | H | H |
| CN | $C_2H_5$ | H | Cl | H | $OCF_2CHCl_2$ | H | Cl |

Tabelle 1 (Fortsetzung):

| R | $R^1$ | $R^2$ | $R^3$ | $R^4$ | $R^5$ | $R^6$ | $R^7$ |
|---|---|---|---|---|---|---|---|
| CN | $C_2H_5$ | H | Br | H | $OCF_2CHCl_2$ | H | H |
| CN | $C_2H_5$ | $CH_3$ | Br | H | $OCF_2CHCl_2$ | H | Br |
| CN | 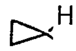 | $C_2H_5$ | Cl | H | $OCF_2CHCl_2$ | H | H |
| CN | 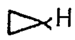 | $C_3H_7$ | Cl | H | $OCF_2CHCl_2$ | H | Cl |
| CN | 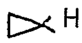 | H | Br | H | $OCF_2CHCl_2$ | H | H |
| CN | 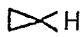 | $CH_3$ | Br | H | $OCF_2CHCl_2$ | H | Br |
| CN | $CH_3$ | $C_2H_5$ | Cl | H | $OCF_2CHF_2$ | H | H |
| CN | $CH_3$ | $C_3H_7$ | Cl | H | $OCF_2CHF_2$ | H | Cl |
| CN | $CH_3$ | H | Br | H | $OCF_2CHF_2$ | H | H |
| CN | $CH_3$ | H | Br | H | $OCF_2CHF_2$ | H | Br |
| CN | $C_2H_5$ | H | Cl | H | $OCF_2CHF_2$ | H | H |
| CN | $C_2H_5$ | H | Cl | H | $OCF_2CHF_2$ | H | Cl |
| CN | $C_2H_5$ | H | Br | H | $OCF_2CHF_2$ | H | H |
| CN | $C_2H_5$ | H | Br | H | $OCF_2CHF_2$ | H | Br |
| CN | 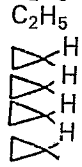 | H | Cl | H | $OCF_2CHF_2$ | H | H |
| CN | | H | Cl | H | $OCF_2CHF_2$ | H | Cl |
| CN | | H | Br | H | $OCF_2CHF_2$ | H | H |
| CN | | H | Br | H | $OCF_2CHF_2$ | H | Br |
| CN | $CH_3$ | $CH_3$ | $CF_3$ | H | $SO_2CH_3$ | H | H |
| CN | $C_2H_5$ | $C_2H_5$ | Br | H | $SO_2CF_3$ | H | H |
| CN | $C_2H_5$ | $C_2H_5$ | Br | H | $SO_2CF_3$ | H | Br |
| CN | $C_2H_5$ | $CH_3$ | $CF_3$ | H | $SO_2CF_3$ | H | H |
| CN | 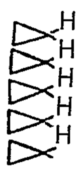 | $CH_3$ | Cl | H | $SO_2CF_3$ | H | H |
| CN | | $CH_3$ | Cl | H | $SO_2CF_3$ | H | Cl |
| CN | | H | Br | H | $SO_2CF_3$ | H | H |
| CN | | H | Br | H | $SO_2CF_3$ | H | Br |
| CN | | H | $CF_3$ | H | $SO_2CF_3$ | H | H |
| CN | $CH_3$ | H | F | H | $SCCl_2F$ | H | H |
| CN | $CH_3$ | H | F | H | $SCCl_2F$ | H | F |
| CN | $CH_3$ | H | F | F | $SCCl_2F$ | F | F |
| CN | $CH_3$ | $CH_3$ | Cl | H | $SCCl_2F$ | H | H |
| CN | $CH_3$ | $CH_3$ | Cl | H | $SCCl_2F$ | H | Cl |
| CN | $CH_3$ | $CH_3$ | Cl | H | $SCCl_2F$ | H | F |
| CN | $CH_3$ | $C_2H_5$ | Br | H | $SCCl_2F$ | H | H |
| CN | $CH_3$ | $C_2H_5$ | Br | H | $SCCl_2F$ | H | Br |
| CN | $C_2H_5$ | $C_2H_5$ | F | H | $SCCl_2F$ | H | H |
| CN | $C_2H_5$ | $C_2H_5$ | F | H | $SCCl_2F$ | H | F |
| CN | $C_2H_5$ | $C_2H_5$ | F | H | $SCCl_2F$ | F | F |
| CN | $C_2H_5$ | $CH_3$ | Cl | H | $SCCl_2F$ | H | H |
| CN | $C_2H_5$ | $CH_3$ | Cl | H | $SCCl_2F$ | H | Cl |
| CN | $C_2H_5$ | H | Cl | H | $SCCl_2F$ | H | F |
| CN | $C_2H_5$ | H | Br | H | $SCCl_2F$ | H | H |
| CN | $C_2H_5$ | H | Br | H | $SCCl_2F$ | H | Br |
| CN | 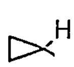 | H | F | H | $SCCl_2F$ | H | H |
| CN | 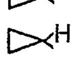 | H | F | H | $SCCl_2F$ | H | F |
| CN | 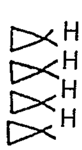 | $CH_3$ | F | F | $SCCl_2F$ | F | F |
| CN | | $CH_3$ | Cl | H | $SCCl_2F$ | H | H |
| CN | | $CH_3$ | Cl | H | $SCCl_2F$ | H | Cl |
| CN | | $C_2H_5$ | Cl | H | $SCCl_2F$ | H | F |

Tabelle 1 (Fortsetzung):

| R | $R^1$ | $R^2$ | $R^3$ | $R^4$ | $R^5$ | $R^6$ | $R^7$ |
|---|---|---|---|---|---|---|---|
| CN | 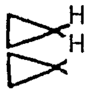 | $C_2H_5$ | Br | H | $SCCl_2F$ | H | H |
| CN | | $C_2H_5$ | Br | H | $SCCl_2F$ | H | Br |
| CN | $CH_3$ | H | F | H | $OCHF_2$ | H | H |
| CN | $CH_3$ | H | F | H | $OCHF_2$ | H | F |
| CN | $CH_3$ | H | F | F | $OCHF_2$ | F | F |
| CN | $CH_3$ | H | Cl | H | $OCHF_2$ | H | H |
| CN | $CH_3$ | H | Cl | H | $OCHF_2$ | H | Cl |
| CN | $CH_3$ | H | Cl | H | $OCHF_2$ | H | F |
| CN | $CH_3$ | H | Br | H | $OCHF_2$ | H | H |
| CN | $CH_3$ | H | Br | H | $OCHF_2$ | H | Br |
| CN | $C_2H_5$ | H | F | H | $OCHF_2$ | H | H |
| CN | $C_2H_5$ | H | F | H | $OCHF_2$ | H | F |
| CN | $C_2H_5$ | H | F | F | $OCHF_2$ | F | F |
| CN | $C_2H_5$ | H | Cl | H | $OCHF_2$ | H | H |
| CN | $C_2H_5$ | H | Cl | H | $OCHF_2$ | H | Cl |
| CN | $C_2H_5$ | H | Cl | H | $OCHF_2$ | H | F |
| CN | $C_2H_5$ | H | Br | H | $OCHF_2$ | H | F |
| CN | $C_2H_5$ | H | Br | H | $OCHF_2$ | H | Br |
| CN | 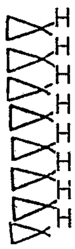 | H | F | H | $OCHF_2$ | H | H |
| CN | | H | F | H | $OCHF_2$ | H | F |
| CN | | H | F | F | $OCHF_2$ | F | F |
| CN | | $CH_3$ | Cl | H | $OCHF_2$ | H | H |
| CN | | $CH_3$ | Cl | H | $OCHF_2$ | H | Cl |
| CN | | $CH_3$ | Cl | H | $OCH_2F$ | H | F |
| CN | | $C_2H_5$ | Br | H | $OCH_2F$ | H | H |
| CN | | $C_2H_5$ | Br | H | $OCHF_2$ | H | Br |
| CN | 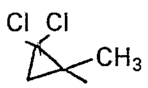 | $C_2H_5$ | Cl | H | $OCF_3$ | H | H |
| CN | 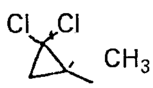 | $C_2H_5$ | Cl | H | $OCF_3$ | H | Cl |
| CN | 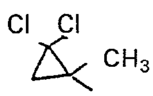 | $C_2H_5$ | Cl | H | $SCF_3$ | H | H |
| CN | 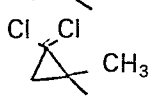 | $C_3H_7$ | Cl | H | $SCF_3$ | H | Cl |
| CN | 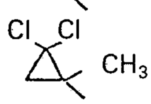 | $C_3H_7$ | Cl | H | $SO_2CF_3$ | H | H |
| CN | 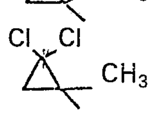 | H | Cl | H | $SO_2CF_3$ | H | Cl |
| CN | 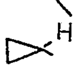 | H | Br | H | $OCH_2CF_3$ | H | Br |
| CN | $CH_3OCH_2$ | H | Cl | H | $OCH_2CF_3$ | H | H |
| CN | $CH_3OCH_2$ | H | Cl | H | $OCH_2CF_3$ | H | Cl |
| CN | $CH_3OCH_2$ | H | Br | H | $OCH_2CF_3$ | H | H |
| CN | $CH_3OCH_2$ | H | Br | H | $OCH_2CF_3$ | H | Br |
| CN | $CH_3$ | H | $SCF_3$ | H | Cl | H | H |
| CN | $C_2H_5$ | H | $SCF_3$ | H | Cl | H | H |
| CN | $CH_3$ | H | $SCF_3$ | H | Cl | H | Cl |
| CN | $C_2H_5$ | H | $SCF_3$ | H | Cl | H | Cl |
| CN | $CH_3$ | H | Cl | H | $SO_2CH_2CF_3$ | H | Cl |
| CN | $CH_3$ | H | Cl | H | $SO_2CH_2CF_3$ | H | Cl |

Tabelle 1 (Fortsetzung):

| R | $R^1$ | $R^2$ | $R^3$ | $R^4$ | $R^5$ | $R^6$ | $R^7$ |
|---|---|---|---|---|---|---|---|
| CN | $C_2H_5$ | H | Cl | H | $SO_2CH_2CF_3$ | H | Cl |
| CN | $C_2H_5$ | H | Cl | H | $SO_2CH_2CF_3$ | H | Cl |
| CN | $CH_3$ | H | F | H | $SCF_3$ | H | H |
| CN | $CH_3$ | H | F | H | $SCF_3$ | H | F |
| CN | $CH_3$ | H | F | F | $SCF_3$ | F | F |
| CN | $CH_3$ | H | Cl | H | $SCF_3$ | H | H |
| CN | $CH_3$ | H | Cl | H | $SCF_3$ | H | Cl |
| CN | $CH_3$ | H | Cl | Cl | $SCF_3$ | H | H |
| CN | $CH_3$ | H | Cl | H | $SCF_3$ | H | F |
| CN | $CH_3$ | H | Br | H | $SCF_3$ | H | H |
| CN | $CH_3$ | H | Br | H | $SCF_3$ | H | Br |
| CN | $C_2H_5$ | H | F | H | $SCF_3$ | H | H |
| CN | $C_2H_5$ | H | F | H | $SCF_3$ | H | F |
| CN | $C_2H_5$ | H | F | H | $SCF_3$ | F | F |
| CN | n-$C_3H_7$ | H | Cl | H | $OCF_3$ | H | H |
| CN | n-$C_3H_7$ | H | Cl | H | $OCF_3$ | H | Cl |
| CN | i-$C_3H_7$ | H | Cl | H | $OCF_3$ | H | H |
| CN | i-$C_3H_7$ | H | Cl | H | $OCF_3$ | H | Cl |
| CN | $C_2H_5$ | $C_2H_5$ | Cl | H | $OCF_3$ | H | H |
| CN | $C_2H_5$ | $C_2H_5$ | Cl | H | $OCF_3$ | H | Cl |
| CN | $CH_3$ | $CH_3$ | Cl | H | $OCF_3$ | H | H |
| CN | $CH_3$ | $CH_3$ | Cl | H | $OCF_3$ | H | Cl |
| CN | n-$C_3H_7$ | $C_3H_7$ | Cl | H | $OCF_3$ | H | H |
| CN | n-$C_3H_7$ | $C_3H_7$ | Cl | H | $OCF_3$ | H | Cl |
| CN | n-$C_3H_7$ | H | Cl | H | $SCF_3$ | H | H |
| CN | n-$C_3H_7$ | H | Cl | H | $SCF_3$ | H | Cl |
| CN | i-$C_3H_7$ | H | Cl | H | $SCF_3$ | H | H |
| CN | i-$C_3H_7$ | H | Cl | H | $SCF_3$ | H | Cl |
| CN | $C_2H_5$ | $C_2H_5$ | Cl | H | $SCF_3$ | H | H |
| CN | $C_2H_5$ | $C_2H_5$ | Cl | H | $SCF_3$ | H | Cl |
| CN | $CH_3$ | $CH_3$ | Cl | H | $SCF_3$ | H | H |
| CN | $CH_3$ | $CH_3$ | Cl | H | $SCF_3$ | H | Cl |
| CN | n-$C_3H_7$ | $C_3H_7$ | Cl | H | $SCF_3$ | H | H |
| CN | n-$C_3H_7$ | $C_3H_7$ | Cl | H | $SCF_3$ | H | Cl |
| CN | n-$C_3H_7$ | H | Cl | H | $SO_2CF_3$ | H | H |
| CN | n-$C_3H_7$ | H | Cl | H | $SO_2CF_3$ | H | Cl |
| CN | i-$C_3H_7$ | H | Cl | H | $SO_2CF_3$ | H | H |
| CN | i-$C_3H_7$ | H | Cl | H | $SO_2CF_3$ | H | Cl |
| CN | $C_2H_5$ | $C_2H_5$ | Cl | H | $SO_2CF_3$ | H | H |
| CN | $C_2H_5$ | $C_2H_5$ | Cl | H | $SO_2CF_3$ | H | Cl |
| CN | n-$C_3H_7$ | $C_3H_7$ | Cl | H | $SO_2CF_3$ | H | H |
| CN | n-$C_3H_7$ | $C_3H_7$ | Cl | H | $SO_2CF_3$ | H | Cl |
| CN | n-$C_3H_7$ | $-\underset{\underset{O}{\|}}{C}-CH_3$ | Cl | H | $OCF_3$ | H | H |
| CN | $C_2H_5$ | $-\underset{\underset{O}{\|}}{C}-CH_3$ | Cl | H | $OCF_3$ | H | Cl |
| CN | n-$C_3H_7$ | $-\underset{\underset{O}{\|}}{C}-CH_3$ | Cl | H | $SCH_3$ | H | Cl |
| CN | n-$C_3H_7$ | $-\underset{\underset{O}{\|}}{C}-CH_3$ | Cl | H | $SO_2CF_3$ | H | Cl |
| CN | n-$C_3H_7$ | $-\underset{\underset{O}{\|}}{C}-CH_3$ | Cl | H | $OCF_3$ | H | Cl |

Verwendet man als Ausgangsstoffe beispielsweise 4-Cyano-5-propionylamino-1-(2,6-dichlor-4-trifluormethylthiophenyl)-pyrazol und Methyljodid, so lässt sich der Reaktionsablauf des erfindunqsgemässen Verfahrens (a) durch das folgende Formelschema darstellen:

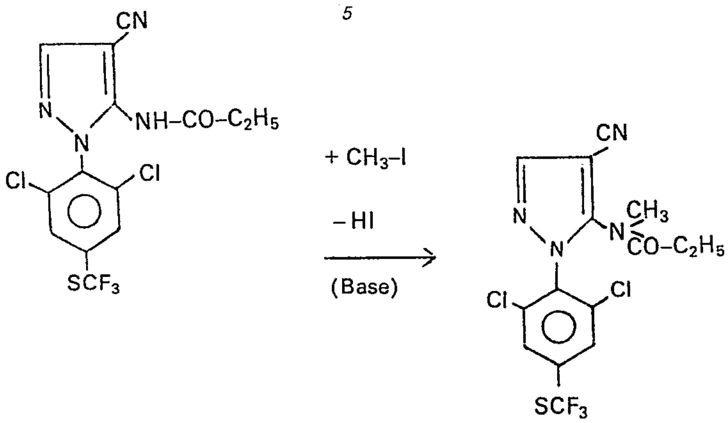


Verwendet man als Ausgangsstoffe beispielsweise 4-Cyano-5-(N-n-propyl-N-propionylamino)- 1-(2-chlor-4-trifluormethoxyphenyl)-pyrazol und Natronlauge, so lässt sich der Reaktionsablauf des erfindungsgemässen Verfahrens (b) durch das folgende Formelschema darstellen:

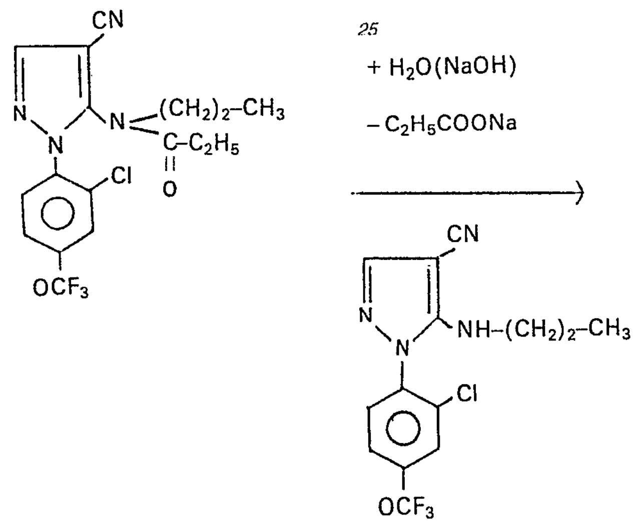


Verwendet man als Ausgangsstoff beispielsweise 4-Cyano-5-isopropylidenimino-1-(2-chlor-4-trifluormethylthiophenyl)-pyrazol und Natriumborhydrid als Reduktionsmittel, so lässt sich der Reaktionsablauf des erfindungsgemässen Verfahrens (c) durch das folgende Formelschema darstellen:

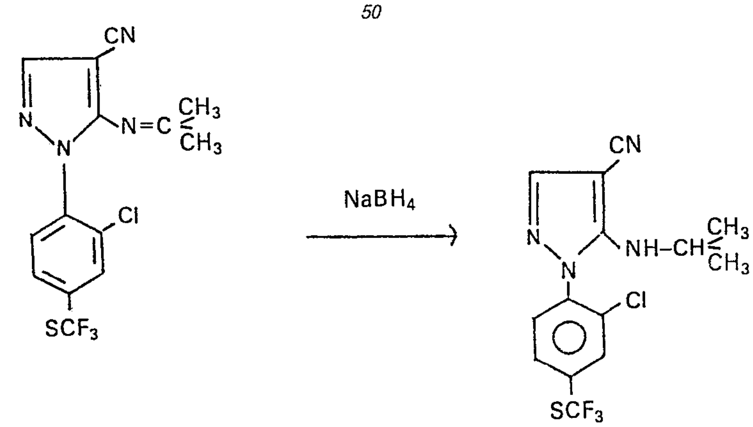

Die zur Durchführung des erfindungsgemässen Verfahrens (a) als Ausgangsstoffe benötigten 5-Amino-pyrazole sind durch die Formel (II) allgemein definiert.

In dieser Formel (II) stehen, R, $R^3$, $R^4$, $R^5$, $R^6$ und $R^7$ vorzugsweise für diejenigen Reste, die bereits im Zusammenhang mit der Beschreibung der erfindungsgemässen Stoffe der Formel (I) als bevorzugt für diese Substituenten genannt wurden. $R^{10}$ steht vorzugsweise für Wasserstoff oder für einen Rest $-\underset{\underset{X}{\|}}{C}-R^8$, wobei X und $R^8$ die gleiche vorzugsweise Bedeutung besitzen, die bereits bei der Beschreibung der erfindungsgemässen Stoffe der Formel (I) für diese Reste genannt wurde.

Die 5-Aminopyrazole der Formel (II) sind teilweise bekannt (vgl. z.B. DE-OS 3 226 496, DE-OS 3 129 429 und EP 34 945).

Man erhält sie nach prinzipiell bekannten Verfahren, beispielsweise wenn man Acrylnitril-Derivate der Formel (V),

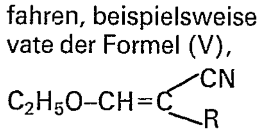

(V)

in welcher

R die oben angegebene Bedeutung hat, mit Phenylhydrazinen der Formel (VI),

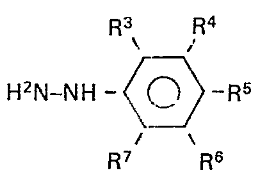

(VI)

in welcher

$R^3$, $R^4$, $R^5$, $R^6$ und $R^7$ die oben angegebene Bedeutung haben, entweder zunächst in einer ersten Stufe, gegebenenfalls in Gegenwart eines Verdünnungsmittels (wie beispielsweise Eisessig oder Ethanol) sowie gegebenenfalls in Gegenwart eines Reaktionshilfsmittels (wie beispielsweise Natriumacetat) bei Temperaturen zwischen −20 °C und +20 °C umsetzt zu den Phenylhydrazin-Derivaten der Formel (VII),

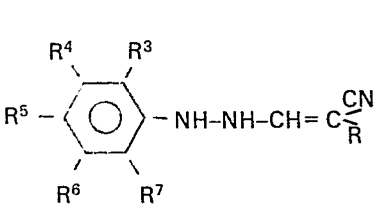

(VII)

in welcher

R, $R^3$, $R^4$, $R^5$, $R^6$ und $R^7$ die oben angegebene Bedeutung haben, und diese in einer zweiten Stufe, gegebenenfalls in Gegenwart eines Verdünnungsmittels (wie beispielsweise Ethylenglykolmonoethylether) bei Temperaturen zwischen +50 °C und +150 °C cyclisiert, oder in einem Reaktionsschritt ohne Isolierung der Zwischenstufe der Formel (VII), gegebenenfalls in Gegenwart eines Verdünnungsmittels (wie beispielsweise Ethylenglykolmonoethylether oder Ethanol) bei Temperaturen zwischen +50 °C und +150 °C direkt cyclisiert und die so erhältlichen 5-Aminopyrazole der Formel (IIa),

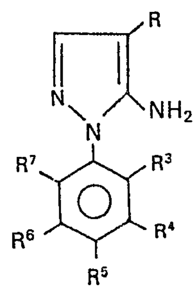

(IIa)

in welcher

R, $R^3$, $R^4$, $R^5$, $R^6$ und $R^7$ die oben angegebene Bedeutung haben, gegebenenfalls mit einem Acylierungsmittel der Formel (VIII),

$$R^8-\underset{\underset{X}{\|}}{C}-A' \qquad (VIII)$$

in welcher

$R^8$ und X die oben angegebene Bedeutung haben und

A' für eine elektronenziehende Austrittsgruppe, wie beispielsweise Halogen oder den Rest $R^8-\underset{\underset{O}{\|}}{C}-O-$, steht,oder mit einem Iso(thio)cyanat der Formel (IX)

$$R^{8'}-N=C=X \qquad (IX)$$

in welcher

X die oben angegebene Bedeutung hat und

$R^{8'}$ für geradkettiges oder verzweigtes Alkyl mit bis zu 4 Kohlenstoffatomen oder für gegebenenfalls einfach oder mehrfach, gleich oder verschieden durch Halogen, jeweils geradkettiges oder verzweigtes Alkyl, Alkoxy oder Halogenalkyl mit jeweils bis zu 4 Kohlenstoffatomen und im Fall des Halogenalkyl mit bis zu 9 gleichen oder verschiedenen Halogenatomen substituiertes Phenyl steht, gegebenenfalls in Gegenwart eines Verdünnungsmittels (wie beispielsweise Dichlormethan oder Acetonitril) und gegebenenfalls in Gegenwart eines Säurebindemittels (wie beispielsweise Kaliumcarbonat oder Triethylamin) bei Temperaturen zwischen −20 °C und +120 °C acyliert (vgl. auch Patentanmeldungen DE-P 33 37 543,7 vom 15.10.1983, DE-P 34 20 985,9 vom 06.06.1984 bzw. DE-P 34 23 582,5 vom 27.06.1984).

Die Acrylnitril-Derivate der Formel (V) sind bekannt (vgl. z.B. EP 34 945 oder DE-OS 3 129 429).

Die Phenylhydrazine der Formel (VI) sind grösstenteils bekannt oder können nach bekannten

Verfahren in einfacher, analoger Art und Weise hergestellt werden (vgl. z.B. Houben-Weyl, 'Methoden der organischen Chemie', Band X/2, S. 203, Thieme Verlag Stuttgart, 1967), indem man beispielsweise die bekannten Aniline der Formel (X),

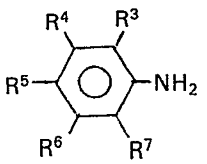

(X)

in welcher

$R^3$, $R^4$, $R^5$, $R^6$ und $R^7$ die oben angegebene Bedeutung haben, mit Natriumnitrit in Gegenwart einer Säure (wie beispielsweise Schwefelsäure) und dann mit Zinn-II-chlorid ebenfalls in Gegenwart einer Säure (wie beispielsweise Salzsäure) bei Temperaturen zwischen −20 °C und +80 °C umsetzt.

Die Acylierungsmittel der Formel (VIII), die Iso(thio)cyanate der Formel (IX) und die Aniline der Formel (X) sind allgemein bekannte Verbindungen der organischen Chemie.

Die weiterhin zur Durchführung des erfindungsgemässen Verfahrens (a) als Ausgangsstoffe benötigten Alkylierungsmittel sind durch die Formel (III) allgemein definiert. In dieser Formel (III) steht $R^1$ vorzugsweise für diejenigen Reste, die bereits bei der Beschreibung der erfindungsgemässen Stoffe der Formel (I) als bevorzugt für diesen Substituenten genannt wurden. A steht vorzugsweise für Halogen, insbesondere für Chlor, Brom oder Jod, oder für jeweils gegebenenfalls substituiertes Alkylsulfonyloxy, Alkoxysulfonyloxy oder Arylsulfonyloxy, wie beispielsweise Methansulfonyloxy, Methoxysulfonyloxy oder p-Toluolsulfonyloxy.

Die Alkylierungsmittel der Formel (III) sind allgemein bekannte Verbindungen der organischen Chemie.

Die zur Durchführung des erfindungsgemässen Verfahrens (b) als Ausgangsstoffe benötigten 5-Acylamino-pyrazole sind durch die Formel (Ia) allgemein definiert. In dieser Formel (Ia) stehen R, $R^1$, $R^3$, $R^4$, $R^5$, $R^6$, $R^7$ und $R^8$ vorzugsweise für diejenigen Reste, die bereits bei der Beschreibung der erfindungsgemässen Stoffe der Formel (I) als bevorzugt für diese Substituenten genannt wurden.

Die 5-Acylamino-pyrazole der Formel (Ia) sind erfindungsgemässe Verbindungen und erhältlich mit Hilfe des erfindungsgemässen Verfahrens (a).

Die zur Durchführung des erfindungsgemässen Verfahrens (c) als Ausgangsstoffe benötigten Azomethine sind durch die Formel (IV) allgemein definiert. In dieser Formel (IV) stehen R, $R^3$, $R^4$, $R^5$, $R^6$ und $R^7$ vorzugsweise für diejenigen Reste, die bereits bei der Beschreibung der erfindungsgemässen Stoffe der Formel (I) als bevorzugt für diese Substituenten genannt wurden, $R^{11}$ steht vorzugsweise für jeweils gegebenenfalls einfach oder mehrfach, gleich oder verschieden substituiertes Methyl, Ethyl, n- oder i-Propyl, n-, i-, s- oder t-Butyl, Allyl, Butenyl, Propargyl oder Butinyl, wobei als Substituenten in Frage kommen: Halogen, Cyano, Hydroxy, Carboxy, sowie jeweils geradkettiges oder verzweigtes Alkoxy oder Alkoxycarbonyl mit jeweils bis zu 6 Kohlenstoffatomen. $R^{12}$ steht unabhängig von $R^{11}$ vorzugsweise für die gleichen Reste wie dieses und ausserdem für Wasserstoff.

Die Azomethine der Formel (IV) sind noch nicht bekannt. Man erhält sie, wenn man 5-Aminopyrazole der Formel (IIa)

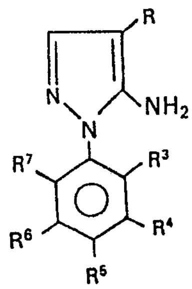

(IIa)

in welcher

R, $R^3$, $R^4$, $R^5$, $R^6$ und $R^7$ die oben angegebene Bedeutung haben, mit Aldehyden oder Ketonen der Formel (XI),

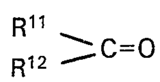

(XI)

in welcher

$R^{11}$ und $R^{12}$ die oben angegebene Bedeutung haben, gegebenenfalls in Gegenwart eines Verdünnungsmittels (wie beispielsweise Methanol oder Ethanol) sowie gegebenenfalls in Gegenwart eines Katalysators (wie beispielsweise Salzsäure oder Schwefelsäure) bei Temperaturen zwischen +20 °C und +120 °C umsetzt.

Die Aldehyde oder Ketone der Formel (XI) sind ebenfalls allgemein bekannte Verbindungen der organischen Chemie.

Als Verdünnungsmittel zur Durchführung des erfindungsgemässen Verfahrens (a) kommen inerte organische Lösungsmittel infrage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemässe Verfahren (a) kann gegebenenfalls auch in einem Zweiphasensystem,

wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumjodid, Tetrabutylammoniumbromid, Tributyl-methylphosphoniumbromid, Trimethyl-$C_{13}/C_{15}$-alkylammoniumchlorid, Dibenzyl-ammoniummethylsulfat, Dimethyl-$C_{12}/C_{14}$-alkyl- benzylammoniumchlorid, Tetrabutylammoniumhydroxid, 15-Krone-5, 18-Krone-6, Triethylbenzylammoniumchlorid, Trimethylbenzylammoniumchlorid.

Als Säurebindemittel zur Durchführung des Herstellungsverfahrens (a) kommen alle üblicherweise verwendbaren anorganischen und organischen Basen infrage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des Herstellungsverfahrens (a) in einem grösseren Bereich variiert werden. Im allgemeinen arbeitet man zwischen −20 °C und +150 °C, vorzugsweise zwischen 0 °C und +100 °C.

Zur Durchführung des Herstellungsverfahrens (a) setzt man pro Mol 5-Amino-pyrazol der Formel (II) im allgemeinen 1,0 bis 20,0 Mol, vorzugsweise 1,0 bis 15,0 Mol an Alkylierungsmittel der Formel (III) und gegebenenfalls 1,0 bis 3,0 Mol, vorzugsweise 1,0 bis 2,0 Mol an Säurebindemittel sowie 0,01 bis 1,0 Mol an Phasentransferkatalysator ein. Die Reaktionsdurchführung, Aufarbeitung und Isolierung der Reaktionsprodukte der Formel (I) erfolgt in allgemein üblicher Art und Weise.

Als Verdünnungsmittel zur Durchführung des erfindungsgemässen Verfahrens (b) kommen ebenfalls inerte organische oder anorganische Lösungsmittel infrage.

Insbesondere seien die bei Verfahren (a) aufgezählten organischen Lösungsmittel genannt. Besonders bevorzugt sind Alkohole wie Methanol oder Ethanol oder deren Gemische mit Wasser.

Das Verfahren (b) wird entweder in Gegenwart einer starken Säure wie beispielsweise Salzsäure, Trifluoressigsäure oder Bromwasserstoffsäure in Eisessig oder in Gegenwart einer Base durchgeführt. Als Basen bevorzugt sind wässrige Lösungen von Natriumhydroxid oder Kaliumhydroxid.

Die Reaktionstemperaturen können bei der Durchführung des erfindungsgemässen Verfahrens (b) in einem grösseren Bereich variiert werden. Im allgemeinen arbeitet man bei Temperaturen zwischen −20 °C und +150 °C, vorzugsweise bei Temperaturen zwischen 0 °C und +120 °C.

Zur Durchführung des erfindungsgemässen Verfahrens (b) setzt man pro Mol 5-Acylamino-pyrazol der Formel (Ia) im allgemeinen 1 bis 30 Mol, vorzugsweise 1 bis 15 Mol, an Säure oder Base ein. Die Reaktionsdurchführung, Aufarbeitung und Isolierung der Reaktionsprodukte der Formel (I) erfolgt in allgemein üblicher Art und Weise.

Als Verdünnungsmittel zur Durchführung des erfindungsgemässen Verfahrens (c) kommen ebenfalls inerte organische Lösungsmittel infrage. Vorzugsweise verwendet man Ether wie beispielsweise Tetrahydrofuran oder Alkohole wie Methanol oder Ethanol.

Als Reduktionsmittel zur Durchführung des erfindungsgemässen Verfahrens (c) kommen komplexe Hydride infrage. Insbesondere verwendet man Alkalimetallborhydride, wie Lithiumborhydrid oder Natriumborhydrid oder Natriumcyanoborhydrid.

Die Reaktionstemperaturen können bei der Durchführung des erfindungsgemässen Verfahrens (c) in einem grösseren Bereich variiert werden. Im allgemeinen arbeitet man bei Temperaturen zwischen −20 °C und +150 °C, vorzugsweise bei Temperaturen zwischen 0 °C und +120 °C.

Zur Durchführung des erfindungsgemässen Verfahrens (c) setzt man pro Mol Azomethin der Formel (IV) im allgemeinen 1 bis 5 Mol, vorzugsweise 1 bis 2 Mol an komplexem Hydrid ein. Die Reaktionsdurchführung, Aufarbeitung und Isolierung der Reaktionsprodukte der Formel (I) erfolgt in allgemein üblicher Art und Weise.

Die erfindungsgemäss verwendbaren Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäss verwendbaren Stoffe als totale oder selektive Herbizide wirken, hängt im wesentlichen von der angewendeten Menge ab.

Die erfindungsgemäss verwendbaren Wirkstoffe können z.B. bei den folgenden Pflanzen verwendet werden:

*Dikotyle Unkräuter der Gattungen:* Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

*Dikotyle Kulturen der Gattungen:* Gossypium, Glycine, Beta, Daucus, Phaseolus, Pisum, Solanum Linum, Ipomoea, Vicia, Nicotiana, Lycopersicon, Arachis, Brassica, Lactuca, Cucumis, Cuburbita.

*Monokotyle Unkräuter der Gattungen:* Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis,

Scirpus, Paspalum, Ischaemum, Sphenoclea, Dactyloctenium, Agrostis, Alopecurus, Apera.

*Monokotyle Kulturen der Gattungen:* Oryza, Zea, Triticum, Hordeum, Avena, Secale, Sorghum, Panicum, Saccharum, Ananas, Asparagus, Allium.

Die Verwendung der erfindungsgemässen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei zeigen die erfindungsgemäss verwendbaren Wirkstoffe der Formel (I) neben einer besonders guten allgemeinherbiziden Wirksamkeit auch eine deutlich verbesserte Kulturpflanzenselektivität in wichtigen Kulturen und können als selektive Unkrautbekämpfungsmittel sowohl in dikotylen Kulturen, wie beispielsweise Baumwollpflanzungen, als auch in monokotylen Kulturen, insbesondere Getreide, wie beispielsweise Weizen oder Hafer, eingesetzt werden. Auch die als Vorprodukte eingesetzten Azomethine der Formel (IV) zeigen herbizide, insbesondere auch selektiv-herbizide Wirksamkeit.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe, wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3- (2,2-dimethylpropyl)-1,3,5-triazin-2,4(1H,3H)-dion oder N-(2-Benzthiazolyl)- N,N'-dimethylharnstoff zur Unkrautbekämpfung in Getreide; 4-Amino-3-methyl-6-phenyl- 1,2,4-triazin-5(4H)-on zu Unkrautbekämpfung in Zuckerrüben und 4-Amino-6- (1,1-dimethylethyl)-3-methylthio-1,2,4-triazin-5(4H)-on zur Unkrautbekämpfung in Sojabohnen, infrage. Auch Mischungen mit N,N-Dimethyl-N'- (3-trifluormethylphenyl)-harnstoff, N,N-Dimethyl-N'- (3-chlor-4-methylphenyl)- harnstoff, N,N-Dimethyl-N'- (4-isopropylphenyl)-harnstoff, 4-Amino-6-t-butyl-3-ethylthio- 1,2,4-triazin-5(4H)-on, 2,4-Dichlorphenoxyessigsäure, 2,4-Dichlorphenoxypropionsäure, (2-Methyl-4-chlorphenoxy)-essigsäure, (4-Chlor-2-methylphenoxy)-propionsäure, Chloressigsäure-N- (methoxymethyl)-2,6-diethylanilid, 2-Ethyl-6-methyl-N- (1-methyl-2-methoxyethyl)- chloracetanilid, 2,6-Dinitro-4-trifluormethyl-N,N-dipropylanilin, Methyl-5- (2,4-dichlorphenoxy)-2- nitrobenzoat; 3,5-Dijod-4-hydroxy-benzonitril; 3-Isopropyl-2,1,3-benzothiadiazin-4-on-2,2-dioxid; 2-Chlor-N- {[(4-methoxy-6-methyl-1,3,5-triazin-2-yl)- amino]-carbonyl}- benzolsulfonamid; 4- Ethylamino-2-t-butylamino-6- methylthio- S-triazin- oder N-(1-Ethylpropyl)-3,4-dimethyl- 2,6- dinitroanilin sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfrass, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Giessen, Spritzen, Sprühen, Streuen.

Die erfindungsgemäss verwendbaren Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem grösseren Bereich schwanken und hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemässen Wirkstoffe geht aus den nachfolgenden Beispielen hervor.

Herstellungsbeispiele:

Beispiel 1

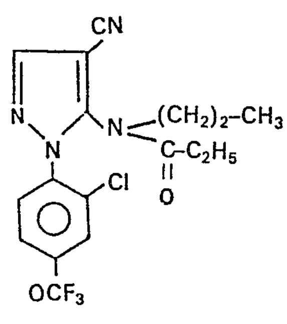

(Verfahren a)

Zu 10,8 g (0,03 Mol) 4-Cyano-5-propionylamino-1- (2-chlor-4-trifluormethoxyphenyl)-pyrazol in 100 ml Tetrahydrofuran gibt man 0,8 g (0,033 Mol) Natriumhydrid und nach beendeter Gasentwicklung 10,2 g (0,06 Mol) n-Propyljodid und rührt 48 Stunden bei Rückflusstemperatur. Zur Aufarbeitung wird im Vakuum eingeengt, der Rückstand in 200 ml Dichlormethan aufgenommen, zweimal mit jeweils 100 ml Wasser gewaschen, über Natriumsulfat getrocknet und im Vakuum eingeengt. Das verbleibende Öl wird säulenchromatographisch gereinigt (Laufmittel: Chloroform/Aceton/9:1) und aus Petrolether kristallisiert. Man erhält 8,0 g (66,6% der Theorie) an 4-Cyano-1-(2-chlor-4-trifluormethoxyphenyl)-5-(N-propyl-N-propionylamino)- pyrazol vom Schmelzpunkt 83 °C.

Herstellung der Ausgangsverbindung:

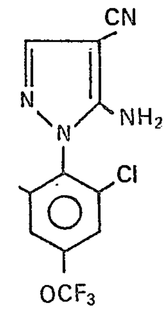

3,08 g (0,25 Mol) Ethoxymethylenmalonsäuredinitril und 5,7 g (0,025 Mol) 2-Chlor-4-trifluormethoxy-phenylhydrazin in 50 ml Ethylenglykolmonoethylether werden 3 Stunden unter Rückfluss erhitzt. Nach dem Abkühlen wird das Reaktionsgemisch auf Wasser gegossen, dann wird der kristalline Niederschlag abgesaugt, mit Petrolether verrührt, gekühlt und abermals abgesaugt.

Man erhält 5,3 g (73,6% der Theorie) an 5-Amino-4-cyano-1- (2-chlor-4-trifluormethoxyphenyl)-pyrazol vom Schmelzpunkt 115 °C.

Beispiel 2

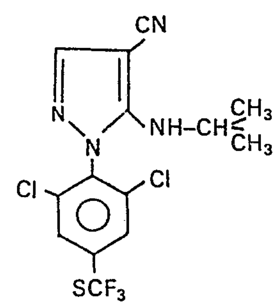

Zu 7,0 g (0,02 Mol) 4-Cyano-5-amino-1-(2,6-dichlor-4-trifluormethylthiophenyl)-pyrazol in 150 ml Tetrahydrofuran gibt man 1,4 g (0,062 Mol) Natriumhydrid und nach beendeter Gasentwicklung 17 g (0,1 Mol) i-Propyljodid und rührt 18 Stunden bei Raumtemperatur. Zur Aufarbeitung wird im Vakuum eingeengt, der Rückstand in 200 ml Dichlormethan aufgenommen, zweimal mit jeweils 100 ml Wasser gewaschen, über Natriumsulfat getrocknet und im Vakuum eingeengt. Der verbleibende Rückstand wird säulenchromatographisch gereinigt (Laufmittel: Chloroform/Aceton = 9:1).

Man erhält 5,2 g (66% der Theorie) an 4-Cyano-1-(2,6-dichlor-4-trifluormethylthio-phenyl)-5-isopropylaminopyrazol vom Schmelzpunkt 130 °C.

Beispiel 3

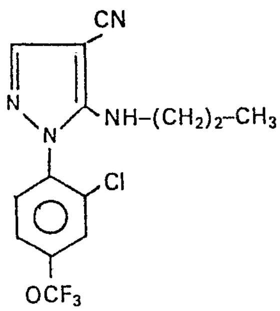

(Verfahren b)

5,0 g (0,012 Mol) 1-(2-Chlor-4-trifluormethoxyphenyl)-4-cyano-5-(N-propionyl-N-n-propylamino)-pyrazol in 200 ml Methanol werden mit 11,6 ml (0,012 Mol) 1-normaler Natronlauge 48 Stunden bei Raumtemperatur gerührt. Zur Aufarbeitung wird im Vakuum eingeengt, der Rückstand in Dichlormethan aufgenommen, zweimal mit jeweils 100 ml Wasser gewaschen, über Natriumsulfat getrocknet, eingeengt und der Rückstand aus Petrolether kristallisiert. Man erhält 3,8 g (92% der Theorie) an 1-(2-Chlor-4-trifluormethoxyphenyl)-4-cyano-5-n-propylamino-pyrazol vom Schmelzpunkt 63 °C.

In entsprechender Weise und gemäss den allgemeinen Herstellungsbeispielen erhält man die folgenden Verbindungen der allgemeinen Formel (I):

Tabelle 2

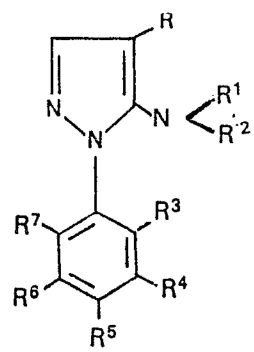

| Bsp. Nr. | R | $R^1$ | $R^2$ | $R^3$ | $R^4$ | $R^5$ | $R^6$ | $R^7$ | Schmelzpunkt (°C) |
|---|---|---|---|---|---|---|---|---|---|
| 4 | CN | $CH_3$ | $CH_3OCO-$ | Cl | H | $-OCF_3$ | H | H | Öl |
| 5 | CN | $CH_3$ | H | Cl | H | $-OCF_3$ | H | H | 165–166 |
| 6 | CN | $C_2H_5$ | $C_2H_5CO-$ | Cl | H | $-OCF_3$ | H | H | 68 |
| 7 | CN | $CH_3$ | $CH_3OCO-$ | Cl | H | $-OCF_3$ | H | Cl | Öl |
| 8 | CN | $C_2H_5$ | H | Cl | H | $-OCF_3$ | H | H | 78 |
| 9 | CN | $CH_3$ | $C_2H_5CO-$ | Cl | H | $-SO_2CF_3$ | H | Cl | 164 |
| 10 | CN | $CH_3$ | $C_2H_5CO-$ | Cl | H | $-OCF_3$ | H | Cl | 108 |
| 11 | CN | $CH_3$ | H | Cl | H | $-OCF_3$ | H | Cl | 157–158 |
| 12 | CN | $CH_3$ | $C_2H_5CO-$ | Cl | H | $-SCF_3$ | H | Cl | 108 |
| 13 | CN | $CH_3$ | H | Cl | H | $-SCF_3$ | H | Cl | 177 |
| 14 | CN | $CH_3$ | H | Cl | H | $-SO_2CF_3$ | H | Cl | 52 |
| 15 | CN | $C_3H_7$ | H | Cl | H | $-SCF_3$ | H | Cl | Öl |
| 16 | CN | $C_3H_7$ | $C_3H_7$ | Cl | H | $-SCF_3$ | H | Cl | Öl |
| 17 | CN | $iC_3H_7$ | H | Cl | H | $-OCF_3$ | H | H | 90 |
| 18 | CN | $iC_3H_7$ | H | Cl | H | $-OCF_3$ | H | Cl | 138 |
| 19 | CN | $C_2H_5$ | H | Cl | H | $-OCF_3$ | H | Cl | 112 |
| 20 | CN | $C_2H_5$ | H | Cl | H | $-SCF_3$ | H | Cl | 114 |
| 21 | CN | $C_2H_5OCOCH_2-$ | $CH_3CO-$ | Cl | H | $-SCF_3$ | H | Cl | 126 |
| 22 | CN | $CH_3OCOCH-$<br>$CH_3$ | H | Cl | H | $-SCF_3$ | H | H | 85–89 |

Anwendungsbeispiele

In den folgenden Anwendungsbeispielen werden die nachstehend aufgeführten Verbindungen als Vergleichssubstanzen eingesetzt:

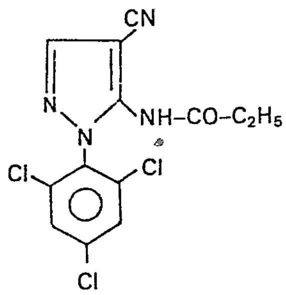

(A)

4-Cyano-5-propionylamino-1-(2,4,6-trichlorphenyl)-pyrazol (bekannt aus DE-OS 3 226 513);

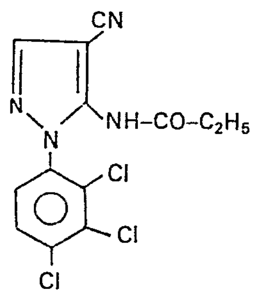

(B)

4-Cyano-5-propionylamino-1-(2,3,4-trichlorphenyl)-pyrazol (bekannt aus DE-OS 3 226 513)

Beispiel

Pre-emergence-Test

Lösungsmittel: 5 Gewichtsteile Aceton

Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmässigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)

100% = totale Vernichtung

In diesem Beispiel zeigt z.B. die Verbindung gemäss Herstellungsbeispiel (5) eine deutliche Überlegenheit in der Nutzpflanzenselektivität im Vergleich zum Stand der Technik; dies gilt insbesondere für Weizen und Baumwolle.

Beispiel

Post-emergence-Test

Lösungsmittel: 5 Gewichtsteile Aceton

Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5–15 cm haben so, dass die jeweils gewünschten Wirkstoffmengen pro Flächeneinheit ausgebracht werden. Die Konzentration der Spritzbrühe wird so gewählt, dass in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0% = keine Wirkung (wie unbehandelte Kontrolle)

100% = totale Vernichtung

In diesem Beispiel zeigt z.B. die Verbindung gemäss Herstellungsbeispiel (3) eine deutliche Überlegenheit in der herbiziden Wirkung, ebenso wie in der Nutzpflanzenselektivität im Vergleich zum Stand der Technik. Dies gilt insbesondere für Hafer.

**Patentansprüche**

1. Substituierte 5-Amino-1-phenylpyrazole der allgemeinen Formel (I)

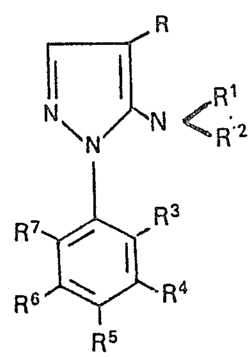

(I)

in welcher

R für Cyano, für Aminocarbonyl oder für jeweils geradkettiges oder verzweigtes Alkoxycarbonyl, Alkenyloxycarbonyl, Alkinyloxycarbonyl, Alkylaminocarbonyl, Alkenylaminocarbonyl, Alkinylaminocarbonyl, Dialkylaminocarbonyl, Dialkenylaminocarbonyl oder Dialkinylaminocarbonyl mit jeweils bis zu 5 Kohlenstoffatomen in den einzelnen aliphatischen Resten steht,

$R^1$ für jeweils gegebenenfalls einfach odermehrfach, gleich oder verschieden substituiertes Alkyl, Alkenyl oder Alkinyl mit jeweils bis zu 8 Kohlenstoffatomen, wobei als Substituenten in Frage kommen: Halogen, Cyano, Nitro, Hydroxy, Carboxy, jeweils geradkettiges oder verzweigtes Alkoxy, oder Alkoxycarbonyl mit jeweils bis zu 6 Kohlenstoffatomen, oder gegebenenfalls durch Alkyl, Alkenyl, Alkinyl, Alkoxy oder Alkylsulfonyl substituiertes Aminocarbonyl, wobei das Stickstoffatom des Aminocarbonylrestes auch Teil eines gesättigten 3- bis 7-gliedrigen Heterocyclus mit bis zu 2 weiteren Heteroatomen, insbesondere Stickstoff, Sauerstoff oder Schwefel, sein kann; ausserdem für gegebenenfalls einfach oder mehrfach, gleich oder verschieden durch Halogen oder geradkettiges oder verzweigtes Alkyl mit 1 bis 4 Kohlenstoffatomen substituiertes Cycloalkyl mit 3 bis 7 Kohlenstoffatomen steht,

$R^2$ unabhängig von $R^1$ für die gleichen Reste wie $R^1$ steht und zusätzlich für Wasserstoff oder für einen Rest $-\underset{X}{\overset{\|}{C}}-R^8$ steht, wobei X für Sauerstoff oder Schwefel steht und

$R^8$ für Wasserstoff, für jeweils geradkettiges oder verzweigtes Alkyl, Alkenyl, Alkinyl, Alkoxyalkyl, Alkylthioalkyl, Alkoxy, Alkylthio, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen, ausserdem für gegebenenfalls einfach oder mehrfach, gleich oder verschieden durch Halogen, niederes Alkyl oder niederes Halogenalkyl substituiertes Cycloalkyl mit 3 bis 7 Kohlenstoffatomen, sowie für jeweils gegebenenfalls einfach oder mehrfach, gleich oder verschieden substituiertes Phenyl, Phenoxy, Phenylthio oder Phenylamino steht, wobei als Phenylsubstituenten infrage kommen: Halogen, jeweils geradkettiges oder verzweigtes Alkyl, Alkoxy oder Halogenalkyl mit jeweils bis zu 4 Kohlenstoffatomen und im Fall des Halogenalkyl mit bis zu 9 gleichen oder verschiedenen Halogenatomen, und

$R^3$, $R^4$, $R^5$, $R^6$ und $R^7$ unabhängig voneinander für Wasserstoff, Fluor, Chlor, Brom, Jod, Nitro, jeweils geradkettiges oder verzweigtes Alkyl, Alkoxy, Alkylsulfonyl oder Alkoxycarbonyl mit bis zu 4 Kohlenstoffatomen in den jeweiligen Alkylteilen oder für einen Rest $-(X')_n-R^9$ stehen, wobei

X' für Sauerstoff, Schwefel, Sulfinyl oder Sulfonyl steht,

n für 0 oder 1 steht und $R^9$ für geradkettiges oder verzweigtes Halogenalkyl mit bis zu 4 Kohlenstoffatomen und bis zu 9 gleichen oder verschiedenen Halogenatomen steht, mit der Massgabe, dass mindestens einer der Reste $R^3$, $R^4$,$R^5$, $R^6$ oder $R^7$ für einen Rest $-(X')_n-R^9$ steht, wobei jedoch nicht gleichzeitig R für Cyano und $R^5$ für Trifluormethyl stehen.

2. Substituierte 5-Amino-1-phenylpyrazole der allgemeinen Formel (I) gemäss Anspruch 1, dadurch gekennzeichnet, dass darin

R für Cyano, Methoxycarbonyl, Ethoxycarbonyl, Allyloxycarbonyl, Propargyloxycarbonyl, Aminocarbonyl, Methylaminocarbonyl, Dimethylaminocarbonyl, Ethylaminocarbonyl, Diethylaminocarbonyl, Diallylaminocarbonyl oder Dipropargylaminocarbonyl steht,

$R^1$ für jeweils gegebenenfalls ein- bis dreifach, gleich oder verschieden substituiertes Methyl, Ethyl, n- oder i-Propyl, n-, i-, s- oder t-Butyl, Allyl, Butenyl, Propargyl oder Butinyl steht, wobei als Substituenten in Frage kommen: Fluor, Chlor, Brom, Jod, Cyano, Nitro, Hydroxy, Carboxy, Methoxy, Ethoxy, n- oder i-Propoxy, Methoxycarbonyl, Ethoxycarbonyl, n- oder i-Propoxycarbonyl, n-, i-, s- oder t-Butoxycarbonyl, Aminocarbonyl, N-Methylaminocarbonyl, N-Ethylaminocarbonyl, N,N-Dimethylaminocarbonyl, N,N-Diethylaminocarbonyl, N,N-Diallylaminocarbonyl, N-Methyl-N-Ethylaminocarbonyl, N-Methyl-N-propylaminocarbonyl, N-Methyl-N-allylaminocarbonyl, N-Methyl-N-propargylaminocarbonyl, N-Methyl-N-methoxyaminocarbonyl, N-Methylsulfonylaminocarbonyl, N-Ethylsulfonylaminocarbonyl, Pyrrolidin-1-ylcarbonyl, Piperidin-1-ylcarbonyl, Morpholin-4-ylcarbonyl oder Perhydroazepin-1-ylcarbonyl; ausserdem für jeweils gegebenenfalls ein- bis fünffach gleich oder verschieden durch Chlor, Methyl oder Ethyl substituiertes Cyclopropyl, Cyclopentyl, Cyclohexyl oder Cycloheptyl steht,

$R^2$ unabhängig von $R^1$ für die gleichen Reste wie $R^1$ steht und zusätzlich für Wasserstoff oder für einen Rest $-\underset{X}{\overset{\|}{C}}-R^8$ steht, wobei X für Sauerstoff oder Schwefel steht und

$R^8$ für Wasserstoff, Methyl, Ethyl, n- und i-Propyl, Allyl, Propargyl, Butenyl, Methoxethyl, Ethoxymethyl, Methoxyethyl, Ethoxyethyl, Methylthiomethyl, Methoxy, Ethoxy, Methylthio, Ethylthio, Methylamino, Ethylamino, Dimethylamino, Trifluormethyl, Trichlorethyl, Dichlorfluorethyl, Difluorchlorethyl, Chlormethyl, Jodmethyl, Brommethyl, Dichlormethyl, 1-Chlorethyl, 2-Chlorethyl, 2-Bromethyl, Heptafluor-n-propyl, für jeweils gegebenenfalls ein- bis vierfach, gleich oder verschieden durch Fluor, Chlor, Brom, Methyl oder Trifluormethyl substituiertes Cyclopropyl, Cyclopentyl oder Cyclohexyl, sowie für jeweils gegebenenfalls ein- bis dreifach, gleich oder verschieden durch Methyl, Methoxy, Chlor oder Trifluormethyl substituiertes Phenyl, Phenoxy, Phenylthio oder Phenylamino steht, und

$R^3$, $R^4$, $R^5$, $R^6$ und $R^7$ unabhängig voneinander für Wasserstoff, Fluor, Chlor, Brom, Cyano, Nitro, Methyl, Ethyl, n- und i-Propyl, Methoxy, Ethoxy, Methylsulfonyl, Methoxycarbonyl, Ethoxycarbonyl oder für einen Rest $-(X')_n-R^9$ stehen, wobei

X' für Sauerstoff, Schwefel, Sulfinyl oder Sulfonyl steht,

n für 0 oder 1 steht und

$R^9$ für Trifluormethyl, Trichlormethyl, Dichlorfluormethyl, Difluorchlormethyl, Dichlormethyl, Chlormethyl, Difluormethyl, Pentafluorethyl, Tetrafluorethyl, Trifluorchlorethyl, Trifluorethyl, Difluordichlorethyl, Trifluordichlorethyl oder Pentachlorethyl stehen, mit der Massgabe, dass mindestens einer der Reste $R^3$, $R^4$, $R^5$, $R^6$ oder $R^7$ für einen Rest $-(X')_n-R^9$ steht, wobei jedoch nicht gleichzeitig R für Cyano und $R^5$ für Trifluormethyl stehen.

3. Verfahren zur Herstellung von substituierten 5-Amino-1-phenylpyrazolen der allgemeinen Formel (I)

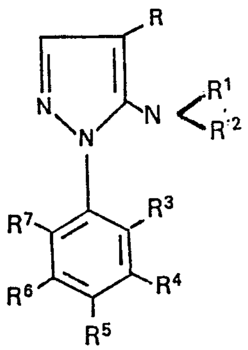

(I)

in welcher

R für Cyano, für Aminocarbonyl oder für jeweils geradkettiges oder verzweigtes Alkoxycarbonyl, Alkenyloxycarbonyl, Alkinyloxycarbonyl, Alkylaminocarbonyl, Alkenylaminocarbonyl, Alkinylaminocarbonyl, Dialkylaminocarbonyl, Dialkenylaminocarbonyl oder Dialkinylaminocarbonyl mit jeweils bis zu 5 Kohlenstoffatomen in den einzelnen aliphatischen Resten steht,

$R^1$ für jeweils gegebenenfalls einfach oder mehrfach, gleich oder verschieden substituiertes Alkyl, Alkenyl oder Alkinyl mit jeweils bis zu 8 Kohlenstoffatomen, wobei als Substituenten in Frage kommen: Halogen, Cyano, Nitro, Hydroxy, Carboxy, jeweils geradkettiges oder verzweigtes Alkoxy, oder Alkoxycarbonyl mit jeweils bis zu 6 Kohlenstoffatomen, oder gegebenenfalls durch Alkyl, Alkenyl, Alkinyl, Alkoxy oder Alkylsulfonyl substituiertes Aminocarbonyl, wobei das Stickstoffatom des Aminocarbonylrestes auch Teil eines gesättigten 3- bis 7-gliedrigen Heterocyclus mit bis zu 2 weiteren Heteroatomen, insbesondere Stickstoff, Sauerstoff oder Schwefel, sein kann; ausserdem für gegebenenfalls einfach oder mehrfach, gleich oder verschieden durch Halogen oder geradkettiges oder verzweigtes Alkyl mit 1 bis 4 Kohlenstoffatomen substituiertes Cycloalkyl mit 3 bis 7 Kohlenstoffatomen steht,

$R^2$ unabhängig von $R^1$ für die gleichen Reste wie $R^1$ steht und zusätzlich für Wasserstoff oder für einen Rest $\underset{X}{\overset{}{-C-}}R^8$ steht, wobei X für Sauerstoff oder Schwefel steht und

$R^8$ für Wasserstoff, für jeweils geradkettiges oder verzweigtes Alkyl, Alkenyl, Alkinyl, Alkoxyalkyl, Alkylthioalkyl, Alkoxy, Alkylthio, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen, ausserdem für gegebenenfalls einfach oder mehrfach, gleich oder verschieden durch Halogen, niederes Alkyl oder niederes Halogenalkyl substituiertes Cycloalkyl mit 3 bis 7 Kohlenstoffatomen, sowie für jeweils gegebenenfalls einfach oder mehrfach, gleich oder verschieden substituiertes Phenyl, Phenoxy, Phenylthio oder Phenylamino steht, wobei als Phenylsubstituenten infrage kommen: Halogen, jeweils geradkettiges oder verzweigtes Alkyl, Alkoxy oder Halogenalkyl mit jeweils bis zu 4 Kohlenstoffatomen und im Fall des Halogenalkyl mit bis zu 9 gleichen oder verschiedenen Halogenatomen, und

$R^3$, $R^4$, $R^5$, $R^6$ und $R^7$ unabhängig voneinander für Wasserstoff, Fluor, Chlor, Brom, Jod, Nitro, jeweils geradkettiges oder verzweigtes Alkyl, Alkoxy, Alkylsulfonyl oder Alkoxycarbonyl mit bis zu 4 Kohlenstoffatomen in den jeweiligen Alkylteilen oder für einen Rest $-(X')_n-R^9$ stehen, wobei

X' für Sauerstoff, Schwefel, Sulfinyl oder Sulfonyl steht,

n für 0 oder 1 steht und $R^9$ für geradkettiges oder verzweigtes Halogenalkyl mit bis zu 4 Kohlenstoffatomen und bis zu 9 gleichen oder verschiedenen Halogenatomen steht, mit der Massgabe, dass mindestens einer der Reste $R^3$, $R^4$, $R^5$, $R^6$ oder $R^7$ für einen Rest-$(X')_n-R^9$ steht, wobei jedoch nicht gleichzeitig R für Cyano und $R^5$ für Trifluormethyl stehen, dadurch gekennzeichnet, dass man

(a) 5-Amino-pyrazole der Formel (II)

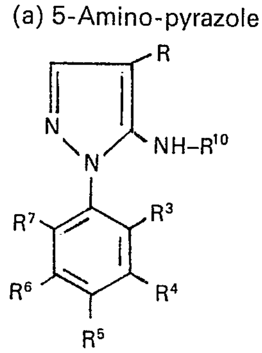

(II)

in welcher

R, $R^3$, $R^4$, $R^5$, $R^6$ und $R^7$ die oben angegebene Bedeutung haben und

$R^{10}$ für Wasserstoff oder für einen Rest $\underset{X}{\overset{}{-C-}}R^8$ steht, wobei X und $R^8$ die oben angegebene Bedeutung haben, mit Alkylierungsmitteln der Formel (III)

$R^1$–A (III)

in welcher

$R^1$ die oben angegebene Bedeutung hat und

A für eine elektronenanziehende Abgangsgruppe steht, gegebenenfalls in Gegenwart eines Verdünnungsmittels und gegebenenfalls in Gegenwart eines Säurebindemittels sowie gegebenenfalls in Gegenwart eines Katalysators umsetzt, oder dass man

(b) 5-Acylaminopyrazole der Formel (Ia)

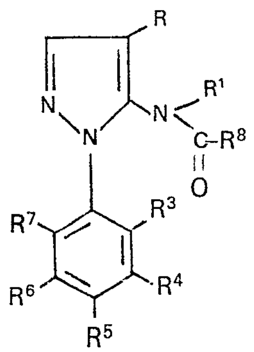

(Ia)

in welcher

R, $R^1$, $R^3$, $R^4$, $R^5$, $R^6$, $R^7$ und $R^8$ die oben angegebene Bedeutung haben, mit Säuren oder Basen, gegebenenfalls in Gegenwart eines Verdünnungsmittels, deacyliert oder dass man

(c) Azomethine der Formel (IV)

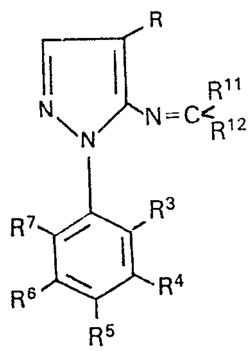

(IV)

in welcher

R, $R^3$, $R^4$, $R^5$, $R^6$ und $R^7$ die oben angegebene Bedeutung haben,

$R^{11}$ für jeweils geradkettiges oder verzweigtes, gegebenenfalls einfach oder mehrfach, gleich oder verschieden substituiertes Alkyl, Alkenyl oder Alkinyl mit jeweils bis zu 7 Kohlenstoffatomen steht, wobei als Substituenten in Frage kommen: Halogen, Cyano, Hydroxy, Carboxy sowie jeweils geradkettiges oder verzweigtes Alkoxy oder Alkoxycarbonyl mit jeweils bis zu 6 Kohlenstoffatomen, und

$R^{12}$ unabhängig von $R^{11}$ für die gleichen Reste wie dieses und ausserdem für Wasserstoff steht, mit einem komplexen Hydrid, gegebenenfalls in Gegenwart eines Verdünnungsmittels reduziert.

4. Herbizide Mittel, gekennzeichnet durch einen Gehalt an mindestens einem substituierten 5-Amino-1-phenylpyrazol der Formel (I) gemäss Anspruch 1 und 3.

5. Verfahren zur Bekämpfung von Unkraut, dadurch gekennzeichnet, dass man substituierte 5-Amino-1-phenylpyrazole der allgemeinen Formel (I) gemäss Anspruch 1 und 3 auf die Unkräuter oder ihren Lebensraum einwirken lässt.

6. Verwendung von substituierten 5-Amino-1-phenylpyrazolen der allgemeinen Formel (I) gemäss Anspruch 1 und 3 zur Bekämpfung von unerwünschtem Pflanzenwachstum.

7. Verfahren zur Herstellung von herbiziden Mitteln, dadurch gekennzeichnet, dass man substituierte 5-Amino-1-phenylpyrazole der allgemeinen Formel (I) gemäss Anspruch 1 und 3 mit Streckmitteln und/oder oberflächenaktiven Mitteln vermischt.

8. Azomethine der Formel (IV)

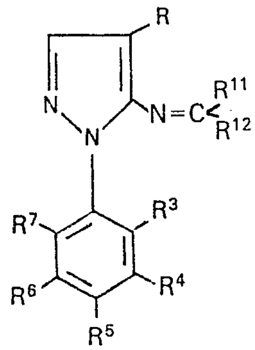

(IV)

in welcher

R, $R^3$, $R^4$, $R^5$, $R^6$ und $R^7$ die in Anspruch 1 angegebene Bedeutung haben,

$R^{11}$ für jweils geradkettiges oder verzweigtes, gegebenenfalls einfach oder mehrfach, gleich oder verschieden substituiertes Alkyl, Alkenyl oder Alkinyl mit jeweils bis zu 7 Kohlenstoffatomen steht, wobei als Substituenten in Frage kommen: Halogen, Cyano, Hydroxy, Carboxy sowie jeweils geradkettiges oder verzweigtes Alkoxy oder Alkoxycarbonyl mit jeweils bis zu 6 Kohlenstoffatomen, und

$R^{12}$ unabhängig von $R^{11}$ für die gleichen Reste wie dieses und ausserdem für Wasserstoff steht.

9. Verfahren zur Herstellung von Azomethinen der Formel (IV)

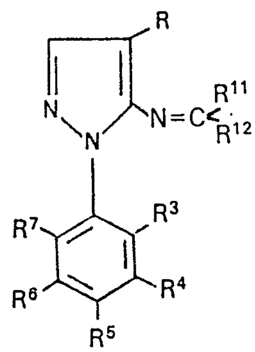

(IV)

in welcher

R, $R^3$, $R^4$, $R^5$, $R^6$ und $R^7$ die in Anspruch 1 angegebene Bedeutung haben,

$R^{11}$ für jeweils geradkettiges oder verzweigtes, gegebenenfalls einfach oder mehrfach, gleich oder verschieden substituiertes Alkyl, Alkenyl oder Alkinyl mit jeweils bis zu 7 Kohlenstoffatomen steht, wobei als Substituenten in Frage kommen: Halogen, Cyano, Hydroxy, Carboxy sowie jeweils geradkettiges oder verzweigtes Alkoxy oder Alkoxycarbonyl mit jeweils bis zu 6 Kohlenstoffatomen, und

$R^{12}$ unabhängig von $R^{11}$ für die gleichen Reste wie dieses und ausserdem für Wasserstoff steht,

dadurch gekennzeichnet, dass man 5-Aminopyrazole der Formel (IIa)

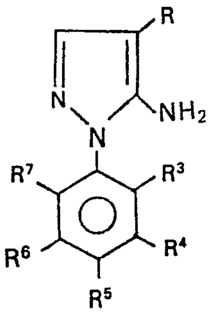

(IIa)

in welcher

R, $R^3$, $R^4$, $R^5$, $R^6$ und $R^7$ die in Anspruch 1 angegebene Bedeutung haben, mit Aldehyden oder Ketonen der Formel (XI)

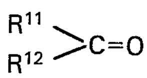

(XI)

in welcher

$R^{11}$ und $R^{12}$ die in Anspruch 3 angegebene Bedeutung haben, gegebenenfalls in Gegenwart eines Verdünnungsmittels und gegebenenfalls in Gegenwart eines Katalysators bei Temperaturen zwischen +20 °C und +120 °C umsetzt.

**Revendications**

1. 5-amino-1-phénylpyrazoles substitués de formule générale (I)

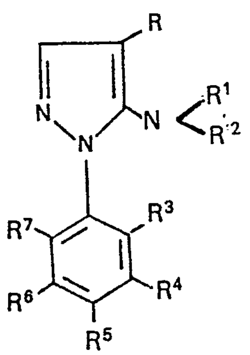

(I)

dans laquelle

R représente un groupe cyano, un groupe amino-carbonyle ou un groupe dialcynylaminocarbonyle, un groupe dialcénylaminocarbonyle, un groupe dialkylaminocarbonyle, un groupe alkynylaminocarbonyle, un groupe alcénylamino-carbonyle, un groupe alkylaminocarbonyle, un groupe alcynaloxycarbonyle, un groupe alcényl-oxycarbonyle ou un groupe alcoxycarbonyle, chaque fois à chaîne droite ou ramifiée et contenant chacun jusqu'à 5 atomes de carbone dans les radicaux aliphatiques individuels,

$R^1$ représente un radical alcynyle, un radical alcényle, ou un radical alkyle, éventuellement substitués chacun, une ou plusieurs fois, de manière identique ou différente, et contenant chacun jusqu'à 8 atomes de carbone, en envisageant, comme substituants: un atome d'halogène, un groupe cyano, un groupe nitro, un groupe hydroxyle, un groupe carboxyle, un groupe alcoxycarbonyle, ou un groupe alcoxy, chacun à chaîne droite ou ramifiée et contenant jusqu'à 6 atomes de carbone, ou un groupe aminocarbonyle éventuellement substitué par un groupe alkyle, un groupe alcényle, un groupe alcynyle, un groupe alcoxy ou par un groupe alkylsulfonyle; l'atome d'azote du radical aminocarbonyle pouvant également faire partie d'un noyau hétérocyclique triangulaire à heptagonal saturé, contenant jusqu'à 2 hétéro-atomes supplémentaires, en particulier, un atome d'azote, un atome d'oxygène ou un atome de soufre; de même que, en outre, un groupe cycloalkyle contenant 3 à 7 atomes de carbone, éventuellement substitués une ou plusieurs fois de manière identique ou différente, par un atome d'halogène ou par un groupe alkyle à chaîne droite ou ramifiée contenant 1 à 4 atomes de carbone,

$R^2$ représente, indépendamment de $R^1$, les mêmes radicaux que ceux mentionnés pour $R^1$ et, en outre, un atome d'hydrogène ou un radical $-\underset{\overset{\|}{X}}{C}-R^8$,

X représentant un atome d'oxygène ou un atome de soufre et

$R^8$ représente un atome d'hydrogéne, un groupe halogénalkyle, un groupe dialkylamino, un groupe alkylamino, un groupe alkylthio, un groupe alcoxy, un groupe alkylthioalkyle, un groupe alcoxyalkyle, un groupe alcynyle, un groupe alcényle ou un groupe alkyle, chacun à chaîne droite ou ramifiée, et contenant chacun jusqu'à 4 atomes de carbone dans les différentes fractions alkyle et contenant, dans le cas du groupe halogénalkyle, jusqu'à 9 atomes d'halogènes identiques ou différents, de même qu'un groupe cycloalkyle contenant 3 à 7 atomes de carbone et éventuellement substitué une ou plusieurs fois de manière identique ou différente par un atome d'halogène, par un groupe alkyle inférieur ou par un groupe halogénalkyle inférieur, ainsi qu'un groupe phénylamino, un groupe phénylthio, un groupe phénoxy ou un groupe phényle éventuellement substitués chaque fois une ou plusieurs fois de manière identique ou différente, en envisageant, comme substituants du groupe phényle: un atome d'halogène, un groupe halogénalkyle, un groupe alcoxy ou un groupe alkyle, chaque fois à chaîne droite ou ramifiée, contenant chacun jusqu'à 4 atomes de carbone et, dans le cas du groupe halogénalkyle, jusqu'à 9 atomes d'halogènes identiques ou différents.

$R^3$, $R^4$, $R^5$, $R^6$ et $R^7$ représentent, indépendamment l'un de l'autre, un atome d'hydrogène, un atome de fluor, un atome de chlore, un atome de brome, un atome d'iode, un groupe nitro; un groupe alcoxycarbonyle, un groupe alkylsulfonyle, un groupe alcoxy ou un groupe alkyle, chaque fois à chaîne droite ou ramifiée, et contenant jusqu'à 4 atomes de carbone dans chacune

des fractions alkyle, ou encore un radical $-(X')_n-R^9$ où

X' représente un atome d'oxygène, un atome de soufre, un groupe sulfynyle ou un groupe sulfonyle,

n représente 0 ou 1 et $R^9$ représente un groupe halogénalkyle à chaîne droite ou ramifiée contenant chacun jusqu'à 4 atomes de carbone et jusqu'à 9 atomes d'halogènes identiques ou différents, avec cette réserve qu'au moins un des radicaux $R^3$, $R^4$, $R^5$, $R^6$ ou $R^7$ représente un radical $-(X')_n-R^9$, R et $R^5$ ne représentant pas toutefois simultanément un groupe cyano et un groupe trifluorométhyle respectivement.

2. 5-Amino-1-phénylpyrazoles substitués de formule générale (I) selon la revendication 1, caractérisés en ce que, dans cette formule:

R représente un groupe cyano, un groupe méthoxycarbonyle, un groupe éthoxycarbonyle, un groupe allyloxycarbonyle, un groupe proparglyoxycarbonyle, un groupe aminocarbonyle, un groupe méthylaminocarbonyle, un groupe diméthylaminocarbonyle, un groupe éthylaminocarbonyle, un groupe diéthylaminocarbonyle, un groupe diallylaminocarbonyle ou un groupe dipropargylaminocarbonyle,

$R^1$ représente un groupe butynyle, un groupe propargyle, un groupe butényle, un groupe allyle, un groupe n-butyle, un groupe i-butyle, un groupe s-butyle, un groupe t-butyle, un groupe n-propyle, un groupe i-propyle, un groupe éthyle ou un groupe méthyle, éventuellement substitués chacun une à trois fois, de manière identique ou différente, en envisageant, comme substituants: un atome de fluor, un atome de chlore, un atome de brome, un atome d'iode, un groupe cyano, un groupe nitro, un groupe hydroxyle, un groupe carboxyle, un groupe méthoxy, un groupe éthoxy, un groupe n-propoxy, un groupe i-propoxy, un grupe méthoxycarbonyle, un groupe éthoxycarbonyle, un groupe n-propoxycarbonyle, un groupe i-propoxycarbonyle, un groupe n-butoxycarbonyle, un groupe i-butoxycarbonyle, un groupe s-butoxycarbonyle, un groupe t-butoxycarbonyle, un groupe aminocarbonyle, un groupe N-méthylaminocarbonyle, un groupe N-éethylaminocarbonyle, un groupe N,N-diméthylaminocarbonyle, un groupe N,N-diéthylaminocarbonyle, un groupe N,N-diallylaminocarbonyle, un groupe N-méthyl-N-éthylaminocarbonyle, un groupe N-méthyl-N-propylaminocarbonyle, un groupe N-méthyl-N-allylaminocarbonyle, un groupe N-méthyl-N-propargylaminocarbonyle, un groupe N-méthyl-N-méthoxyaminocarbonyle, un groupe N-méthylsulfonylaminocarbonyle, un groupe N-éthylsulfonylaminocarbonyle, un groupe pyrolidin-1-ylcarbonyle, un groupe pipéridin-1-ylcarbonyle, un groupe morpholin-4-ylcarbonyle ou un groupe perhydroazepin-1-ylcarbonyle; de même qu'un groupe cycloheptyle, un groupe cyclohexyle, un groupe cyclopentyle ou un groupe cyclopropyle éventuellement substitué une à cinq fois de manière identique ou différente par un atome de chlore, un groupe méthyle ou un groupe éthyle,

$R^2$ représente, indépendamment de $R^1$, les mêmes radicaux que ceux mentionnés pour $R^1$ et, en outre, un atome d'hydrogène ou un radical $-\underset{\underset{X}{\Vert}}{C}-R^8$,

X représentant un atome d'oxygène ou un atome de soufre, et

$R^8$ représente un atome d'hydrogène, un groupe méthyle, un groupe éthyle, un groupe n-propyle, un groupe i-propyle, un groupe allyle, un groupe propargyle, un groupe butényle, un groupe méthoxyméthyle, un groupe éthoxyméthyle, un groupe méthoxyéthyle, un groupe éthoxyéthyle, un groupe méthylthiométhyle, un groupe méthoxy, un groupe éthoxy, un groupe méthylthio, un groupe éthylthio, un groupe méthylamino, un groupe éthylamino, un groupe diméthylamino, un groupe trifluorométhyle, un groupe trichloréthyle, un groupe dichlorofluoroéthyle, un groupe difluorochloroéthyle, un groupe chlorométhyle, un groupe iodométhyle, un groupe bromométhyle, un groupe dichlorométhyle, un groupe 1-chloréthyle, un groupe 2-chloréthyle, un groupe 2-bromoéthyle, un groupe heptafluoro-n-propyle, un groupe cyclohexyle, un groupe cyclopentyle ou un groupe cyclopropyle substitué de manière identique ou différente par un atome de fluor, un atome de chlore, un atome de brome, un groupe méthyle, ou un groupe toifluorométhyle, de même qu'un groupe phénylamino, un groupe phénylthio, un groupe phénoxy ou un groupe phényle éventuellement substitué une à trois fois de manière identique ou differente par un groupe méthyle, un groupe méthoxy, un atome de chlore ou par un groupe trifluorométhyle, et

$R^3$, $R^4$, $R^5$, $R^6$ et $R^7$ représentent chacun, indépendamment l'un de l'autre, un atome d'hydrogène, un atome de fluor, un atome de chlore, un atome de brome, un groupe cyano, un groupe nitro, un groupe méthyle, un groupe éthyle, un groupe n-propyle, un groupe i-propyle, un groupe méthoxy, un groupe éthoxy, un groupe méthylsulfonyle, un groupe méthoxycarbonyle, un groupe éthoxycarbonyle ou un radical $-(X')_n-R^9$,

X' représentant un atome d'oxygène, un atome de soufre, un groupe sulfinyle ou un groupe sulfonyle,

n est égal à 0 ou 1, et

$R^9$ représente un groupe trifluorométhyle, un groupe trichlorométhyle, un groupe dichlorofluorométhyle, un groupe difluorochlorométhyle, un groupe dichlorométhyle, un groupe chlorométhyle, un groupe difluorométhyle, un groupe pentafluoroéthyle, un groupe tétrafluoroéthyle, un groupe trifluorochloroéthyle, un groupe trifluoroéthyle, un groupe difluorodichloréthyle, un groupe trifluorodichloréthyle, un groupe trifluorodichloréthyle ou un groupe pentachloréthyle, avec cette réserve qu'au moins un des radicaux $R^3$, $R^4$, $R^5$, $R^6$ ou $R^7$ représente un radical $-(X')_n-R^9$, R et $R^5$ ne représentant pas toutefois simultanément un groupe cyano et un groupe trifluorométhyle respectivement.

3. Procédé de préparation de 5-amino-1-phénylpyrazoles substitués de formule générale (I)

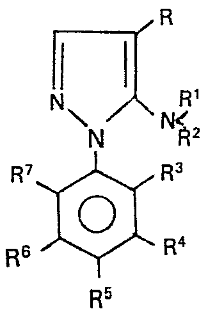

(I)

dans laquelle

R représente un groupe cyano, un groupe aminocarbonyle ou un groupe dialcynylaminocarbonyle, un groupe dialcénylaminocarbonyle, un groupe dialkylaminocarbonyle, un groupe alcynylaminocarbonyle, un groupe alcénylaminocarbonyle, un groupe alkylaminocarbonyle, un groupe alcynyloxycarbonyle, un groupe alcényloxycarbonyle ou un groupe alcoxycarbonyle, chacun à chaîne droite ou ramifiée et contenant chacun jusqu'à cinq atomes de carbone dans les différents radicaux aliphatiques,

$R^1$ représente un groupe alcynyle, un groupe alcényle ou un groupe alkyle, éventuellement substitué une ou plusieurs fois de manière identique ou différente, et contenant chacun jusqu'à huit atomes de carbone, en envisageant, comme substituants: un atome d'halogène, un groupe cyano, un groupe nitro, un groupe hydroxyle, un groupe carboxyle; un groupe alcoxycarbonyle ou un groupe alcoxy chacun à chaîne droite ou ramifiée, contenant chacun jusqu'à six atomes de carbone; ou un groupe aminocarbonyle éventuellement substitué par un groupe alkyle, un groupe alcényle, un groupe alcynyle, un groupe alcoxy ou par un groupe alkylsulfonyle, l'atome d'azote du radical aminocarbonyle pouvant également faire partie d'un noyau hétérocyclique triangulaire à heptagonal contenant jusqu'à deux hétéro-atomes supplémentaires, en particulier, un atome d'azote, un atome d'oxygène ou un atome de soufre; de même qu'un groupe cycloalkyle contenant trois à sept atomes de carbone, et éventuellement substitué une ou plusieurs fois de manière identique ou différente par un atome d'halogène ou un groupe alkyle à chaîne droite ou ramifiée contenant un à quatre atomes de carbone,

$R^2$ représente, indépendamment de $R^1$, les mêmes radicaux que ceux mentionnés pour $R^1$ et, en outre, un atome d'hydrogène ou un radical $-\underset{\underset{X}{\|}}{C}-R^8$,

X représentant un atome d'oxygène ou de soufre, et

où $R^8$ représente un atome d'hydrogéne, un groupe halogénalkyle, un groupe dialkylamino, un groupe alkylamino, un groupe alkylthio, un groupe alcoxy, un groupe alkylthioalkyle, un groupe alcoxyalkyle, un groupe alcynyle, un groupe alcényle ou un groupe alkyle, chacun èchaîne droite ou ramifiée, et contenant chacun jusqu'à quatre atomes de carbone dans les différentes fractions alkyles et, dans le cas du groupe halogénalkyle, jusqu'à neuf atomes d'halogènes identiques ou différents, de même qu'un groupe cycloalkyle contenant trois à sept atomes de carbone et éventuellement substitué une ou plusieurs fois, de manière identique ou différente, par un atome d'halogène, un groupe alkyle inférieur ou un groupe halogénalkyle inférieur, ainsi qu'un groupe phénylamino, un groupe phénylthio, un groupe phénoxy ou un groupe phényle, éventuellement substitués chacun une ou plusieurs fois, de manière identique ou différente, en envisageant, comme substituants du groupe phényle: un atome d'halogène; un groupe halogénalkyle, un groupe alcoxy ou un groupe alkyle, chacun à chaîne droite ou ramifiée, et contenant chacun jusqu'à quatre atomes de carbone et, dans le cas du groupe halogénalkyle, jusqu'à neuf atomes d'halogènes identiques ou différentes, et

$R^3$, $R^4$, $R^5$, $R^6$ et $R^7$ représentent chacun indépendamment l'un de l'autre, un atome d'hydrogène, un atome de fluor, un atome de chlore, un atome de brome, un atome d'iode, un groupe nitro; un groupe alkoxycarbonyle, un groupe alkylsulfonyle, un groupe alcoxy ou un groupe alkyle chacun à chaîne droite ou ramifiée et contenant jusqu'à quatre atomes de carbone dans chaque fraction alkyle, ou un radical $-(X')_n-R^9$,

X' représentant un atome d'oxygène, un atome de soufre, un groupe sulfinyle ou un groupe sulfonyle,

n étant égal à 0 ou 1 et $R^9$ représentant un groupe halogénalkyle à chaîne droite ou ramifiée, contenant jusqu'à 4 atomes de carbone et jusqu'à 9 atomes d'halogènes identiques ou différents, avec cette réserve qu'au moins un des radicaux $R^3$, $R^4$, $R^5$, $R^6$ ou $R^7$ représente un radical $-(X')_n-R^9$, R et $R^5$ ne représentant pas toutefois simultanément un groupe cyano et un groupe trifluorométhyle respectivement, caractérisé en ce que:

(a) on fait réagir des 5-amino-pyrazoles de formule (II)

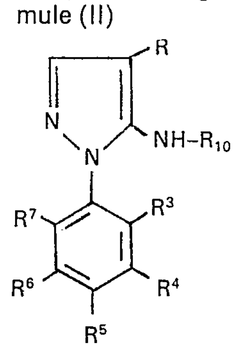

(II)

dans laquelle

R, $R^3$, $R^4$, $R^5$, $R^6$ et $R^7$ ont les significations indiquées ci-dessus, et

$R^{10}$ représente un atome d'hydrogène ou un radical de formule $-\underset{\underset{X}{\|}}{C}-R^8$,

X et $R^8$ ayant les significations indiquées ci-dessus, avec des agents d'alkylation de formule (III)

$R^1$–A (III)

dans laquelle $R^1$ a les significations indiquées ci-dessus, et A représente un groupe qui s'éloigne attirant les électrons, éventuellement en présence d'un diluant et éventuellement en présence d'un agent fixateur d'acide, ainsi qu'éventuellement en présence d'un catalyseur, ou en ce que

(b) on soumet, à une déacylation, des 5-acylaminopyrazoles de formule (Ia)

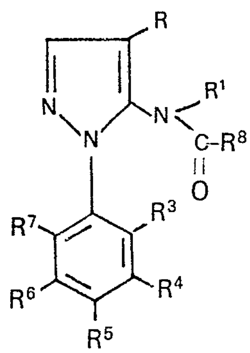

(Ia)

dans laquelle
R, $R^1$, $R^3$, $R^4$, $R^5$, $R^6$, $R^7$ et $R^8$ ont les significations indiquées ci-dessus, avec des acides ou des bases, éventuellement en présence d'un diluant, ou en ce que

(c) on réduit des azométhines de formule (IV)

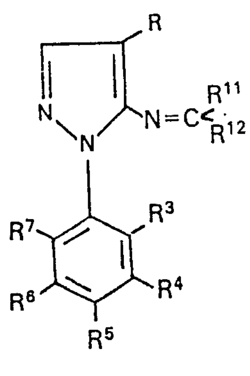

(IV)

dans laquelle
R, $R^3$, $R^4$, $R^5$, $R^6$ et $R^7$ ont les significations indiquées ci-dessus,

$R^{11}$ représente un groupe alcynyle, un groupe alcényle ou un groupe alkyle, contenant chacun jusqu'à 7 atomes de carbone, chacun à chaîne droite ou ramifiée, et éventuellement substitué une ou plusieurs fois, de manière identique ou différente, en envisageant, comme substituants: un atome d'halogène, un groupe cyano, un groupe hydroxyle, un groupe carboxyle, de même qu'un groupe alcoxy ou un groupe alcoxycarbonyle, chacun à chaîne droite ou ramifiée et contenant chacun jusqu'à 6 atomes de carbone et,

$R^{12}$ représente, indépendamment de $R^{11}$, les mêmes radicaux que ceux mentionnés pour celui-ci, et, en outre, un atome d'hydrogène, avec un hydrure complexe, éventuellement en présence d'un diluant.

4. Agents herbicides, caractérisés en ce qu'ils contiennent au moins un 5-amino-1-phénylpyrazole substitué de formule (I) selon les revendications 1 et 3.

5. Procédé en vue de combattre les plantes adventices, caractérisé en ce qu'on fait agir des 5-amino-1-phénylpyrazoles substitués de formule (I) selon les revendications 1 et 3, sur les plantes adventices ou leur biotope.

6. Utilisation de 5-amino-1-phénylpyrazoles substitués de formule générale (I) selon les revendications 1 et 3 en vue de combattre la croissance inopportune des plantes.

7. Procédé de préparation d'agents herbicides, caractérisé en ce qu'on mélange des 5-amino-1-phénylpyrazoles substitués de formule générale (I) selon les revendications 1 et 3, avec des diluants et/ou des agents tensio-actifs.

8. Azométhines de formule (IV)

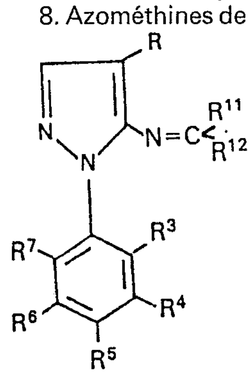

(IV)

dans laquelle
R, $R^3$, $R^4$, $R^5$, $R^6$ et $R^7$ ont les significations indiquées dans la revendication 1,

$R^{11}$ représente un groupe alcynyle, un groupe alcényle ou un groupe alkyle, chacun à chaîne droite ou ramifiée, éventuellement substitué une ou plusieurs fois, de manière identique ou différente, et contenant chacun jusqu'à 7 atomes de carbone, en envisageant, comme substituants: un atome d'halogène, un groupe cyano, un groupe hydroxyle, un groupe carboxyle, de même qu'un groupe alcoxy ou un groupe alcoxycarbonyle, chacun à chaîne droite ou ramifiée, et contenant chacun jusqu'à 6 atomes de carbone, et,

$R^{12}$ représente, indépendamment, de $R^{11}$, les mêmes radicaux que celui-ci et, en outre, un atome d'hydrogène.

9. Procédé de préparation d'azométhines de formule (IV)

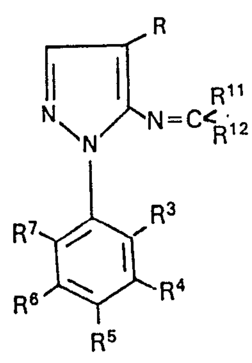

(IV)

dans laquelle

R, $R^3$, $R^4$, $R^5$, $R^6$ et $R^7$ ont les significations indiquées dans la revendication 1,

$R^{11}$ représente un groupe alcynyle, un groupe alcényle ou un groupe alkyle, chacun à chaîne droite ou ramifiée, éventuellement substitué une ou plusieurs fois, de manière identique ou différente, et contenant chacun jusqu'à 7 atomes de carbone, en envisageant, cme substituants: un atome d'halogène, un groupe cyano, un groupe hydroxyle, un groupe carboxyle, de même qu'un groupe alkoxycarbonyle ou un groupe alkoxy, chacun à chaîne droite ou ramifiée, et contenant chacun jusqu'à 6 atomes de carbone, et

$R^{12}$ représente, indépendamment de $R^{11}$, les mêmes radicaux que celui-ci et, en outre, un atome d'hydrogène, caractérisé en ce qu'on fait réagir des 5-aminopyrazoles de formule (IIa)

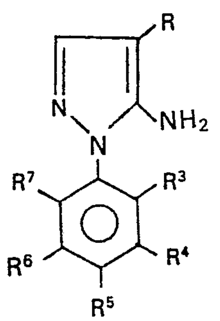

(IIa)

dans laquelle

R, $R^3$, $R^4$, $R^5$, $R^6$ et $R^7$ ont les significations indiquées dans la revendication 1, avec des aldéhydes ou des cétones de formule (XI)

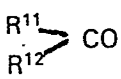

(XI)

dans laquelle

$R^{11}$ et $R^{12}$ ont les significations indiquées dans la revendication 3, éventuellement en présence d'un diluant et éventuellement en présence d'un catalyseur, à des températures comprises entre +20 °C et +120 °C.

## Claims

1. Substituted 5-amino-1-phenylpyrazoles of the general formula (I)

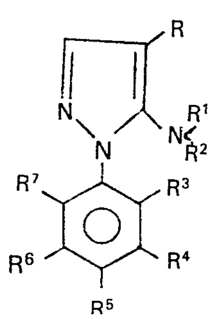

(I)

in which

R represents cyano or aminocarbonyl, or represents alkoxycarbonyl, alkenyloxycarbonyl, alkinylloxycarbonyl, alkylaminocarbonyl, alkenylaminocarbonyl, alkinylaminocarbonyl, dialkylaminocarbonyl, dialkenylaminocarbonyl or dialkinylaminocarbonyl, each of which has up to 5 carbon atoms in the individual aliphatic radicals and each of which is straight-chain or branched, $R^1$ represents alkyl, alkenyl or alkinyl, each of which has up to 8 carbon atoms and each of which is optionally monosubstituted or polysubstituted by identical or different substituents, possible substituents being: halogen, cyano, nitro, hydroxyl and carboxyl, alkoxy and alkoxycarbonyl, each of which has up to 6 carbon atoms and each of which is straight-chain or branched, and aminocarbonyl which is optionally substituted by alkyl, alkenyl, alkinyl, alkoxy or alkylsulphonyl, it being possible for the nitrogen atom of the aminocarbonyl radical also to be part of a saturated 3-membered to 7-membered heterocyclic radical with up to 2 further hetero-atoms, in particular nitrogen, oxygen or sulphur; or represents cycloalkyl which has 3 to 7 carbon atoms and is optionally monosubstituted or polysubstituted by identical or different radicals from the group comprising halogen and straight-chain or branched alkyl with 1 to 4 carbon atoms, $R^2$ independently of $R^1$ represents the same radicals as $R^1$, and also represents hydrogen or a radical $-\underset{\underset{X}{\|}}{C}-R^8$, wherein

X represents oxygen or sulphur and

$R^8$ represents hydrogen, or represents alkyl, alkenyl, alkinyl, alkoxyalkyl, alkylthioalkyl, alkoxy, alkylthio, alkylamino, dialkylamino or halogenalkyl, each of which has up to 4 carbon atoms in the individual alkyl parts and each of which is straight-chain or branched, halogenoalkyl having up to 9 identical or different halogen atoms, or represents cycloalkyl which has 3 to 7 carbon atoms and is optionally monosubstituted or polysubstituted by identical or different substituents from the group comprising halogen, lower alkyl and lower halogenalkyl, or represents phenyl, phenoxy, phenylthio or phenylamino, each of which is optionally monosubstituted or polysubstituted by identical or different substituents, possible substituents of the phenyl being: halogen and alkyl, alkoxy and halogenoalkyl, each of which has up to 4 carbon atoms and each of which is straight-chain or branched, halogenoalkyl having up to 9 identical or different halogen atoms, and $R^3$, $R^4$, $R^5$, $R^6$ and $R^7$ independently of one another represent hydrogen, fluorine, chlorine, bromine, jodine or nitro, or alkyl, alkoxy, alkylsulphonyl or alkoxycarbonyl, each of which has up to 4 carbon atoms in the particular alkyl parts and each of which is straight-chain or branched, or represent a radical $-(X')_n-R^9$, wherein

X' represents oxygen, sulphur, sulphinyl or sulphonyl,

n represents 0 or 1 and

$R^9$ represents straight-chain or branched halogenoalkyl with up to 4 carbon atoms and up to 9 identical or different halogen atoms, with the proviso that at least one of the radicals $R^3$, $R^4$, $R^5$, $R^6$ or $R^7$ represents a radical $-(X')_n-R^9$, but, at the same time, R does not represent cyano and $R^5$ does not represent trifluoromethyl.

2. Substituted 5-amino-1-phenylpyrazoles of the general formula (I) according to Claim 1, characterized in that, in this formula,

R represents cyano, methoxycarbonyl, ethoxycarbonyl, allyloxycarbonyl, propargyloxycarbonyl, aminocarbonyl, methylaminocarbonyl, dimethylaminocarbonyl, ethylaminocarbonyl, diethylaminocarbonyl, diallyalminocarbonyl or dipropargylaminocarbonyl,

$R^1$ represents methyl, ethyl, n- or i-propyl, n-, i-, s- or t-butyl, allyl, butenyl, propargyl or butinyl, each of which is optionally mono-, di- or tri-substituted by identical or different substituents, possible substituents being: fluorine, chlorine, bromine, jodine, cyano, nitro, hydroxyl, carboxyl, methoxy, ethoxy, n- or i-propoxy, methoxycarbonyl, ethoxycarbonyl, n- or i-propoxycarbonyl, n-, i-, s- or t-butoxycarbonyl, aminocarbonyl, N-methylaminocarbonyl, N-ethylaminocarbonyl, N,N-dimethylaminocarbonyl, N,N-diethylaminocarbonyl, N,N-diallylaminocarbonyl, N-methyl-N-ethylaminocarbonyl, N-methyl-N-propylaminocarbonyl, N-methyl-N-allylaminocarbonyl, N-methyl-N-propargylaminocarbonyl, N-methyl-N-methoxyaminocarbonyl, N-methylsulphonylaminocarbonyl, N-ethylsulphonylaminocarbonyl, pyrrolidin-1-ylcarbonyl, piperidin-1-ylcarbonyl, morpholin-4-ylcarbonyl and perhydroazepin-1-yl-carbonyl; or represents cyclopropyl, cyclopentyl, cyclohexyl or cycloheptyl, each of which is optionally mono-, di-, tri-, tetra- or pentasubstituted by identical or different substituents from the group comprising chlorine, methyl and ethyl,

$R^2$ idenpendently of $R^1$ represents the same radicals as $R^1$, and additionally represents hydrogen or a radical $-\underset{\overset{\|}{X}}{C}-R^8$,

wherein

X represents oxygen or sulphur and

$R^8$ represents hydrogen, methyl, ethyl, n- or i-propyl, allyl, propargyl, butenyl, methoxymethyl, ethoxymethyl, methoxyethyl, ethoxyethyl, methylthiomethyl, methoxy, ethoxy, methylthio, ethylthio, methylamino, ethylamino, dimethylamino, trifluoromethyl, trichloroethyl, dichlorofluoroethyl, difluorochloroethyl, chloromethyl, jodomethyl, bromomethyl, dichloromethyl, 1-chloroethyl, 2-chloroethyl, 2-bromoethyl or heptafluoro-n-propyl, or represents cyclopropyl, cyclopentyl or cyclohexyl, each of which is optionally mono-, di-, tri- or tetra-substituted by identical or different substituents from the group comprising fluorine, chlorine, bromine, methyl and trifluoromethyl, or represents phenyl, phenoxy, phenylthio or phenylamino, each of which is optionally mono-, di- or tri-substituted by identical or different substituents from the group comprising methyl, methoxy, chlorine and trifluoromethyl, and

$R^3$, $R^4$, $R^5$, $R^6$ and $R^7$ independently of one another represent hydrogen, fluorine, chlorine, bromine, cyano, nitro, methyl, ethyl, n- or i-propyl, methoxy, ethoxy, methylsulphonyl, methoxycarbonyl or ethoxycarbonyl, or represent a radical $-(X')_n-R^9$, wherein

X' represents oxygen, sulphur, sulphinyl or sulphonyl,

n represents 0 or 1 and

$R^9$ represents trifluoromethyl, trichloromethyl, dichlorofluoromethyl, difluorochloromethyl, dichloromethyl, chloromethyl, difluoromethyl, pentafluoroethyl, tetrafluoroethyl, trifluorochloroethyl, trifluoroethyl, difluorodichloroethyl, trifluorodichloroethyl or pentachloroethyl, with the proviso that at least one of the radicals $R^3$, $R^4$, $R^5$, $R^6$ or $R^7$ represents a radical $-(X')_n-R^9$, but, at the same time, R does not represent cyano and $R^5$ does not represent trifluoromethyl,

3. Process for the preparation of substituted 5-amino-1-phenylpyrazoles of the general formula (I) in which

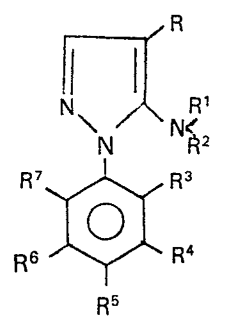

(I)

R represents cyano or aminocarbonyl, or represents alkoxycarbonyl, alkenyloxycarbonyl,alkinyloxycarbonyl, alkylaminocarbonyl, alkenylaminocarbonyl, alkinylaminocarbonyl, dialkylaminocarbonyl, dialkenylaminocarbonyl or dialkinylaminocarbonyl, each of which has up to 5 carbon ats in the individual aliphatic radicals and each of which is straight-chain or branched,

$R^1$ represents alkyl, alkenyl or alkinyl, each of which has up to 8 carbon atoms and each of which is optionally monosubstituted or polysubstituted by identical or different substituents, possible substituents being: halogen, cyano, nitro, hydroxyl and carboxyl, alkoxy and alkoxycarbonyl, each of which has up to 6 carbon atoms and each of which is straight-chain or branched, and aminocarbonyl which is optionally substituted by alkyl, alkenyl, alkinyl, alkoxy or alkylsulphonyl, it being possible for the nitrogen atom of the aminocarbonyl radical also to be part of a saturated 3-membered to 7-membered heterocyclic radical with up to 2 further hetero-atoms, in particular nitrogen, oxygen or sulphur; or represents cycloalkyl which has 3 to 7 carbon atoms and is optionally monosubstituted or polysubstituted by identical or different radicals from the group comprising halogen and straight-chain or branched alkyl with 1 to 4 carbon atoms,

$R^2$ independently of $R^1$ represents the same radicals as $R^1$, and also represents hydrogen or a radical $-\underset{\underset{X}{\|}}{C}-R^8$, wherein

X represents oxygen or sulphur and

$R^8$ represents hydrogen, or represents alkyl, alkenyl, , alkinyl, alkoxyalkyl, alkylthioalkyl, alkoxy, alkylthio, alkylamino, dialkylamino or halogenoalkyl, each of which has up to 4 carbon atoms in the individual alkyl parts and each of which is straight-chain or branched, halogenoalkyl having up to 9 identical or different halogen atoms, or represents cycloalkyl which has 3 to 7 carbon atoms and is optionally monosubstituted or polysubstituted by identical or different substituents from the group comprising halogen, lower alkyl and lower halogenoalkyl, or represents phenyl, phenoxy, phenylthio or phenylamino, each of which is optionally monosubstituted or polysubstituted by identical or different substituents, possible substituents of the phenyl being: halogen and alkyl, alkoxy and halogenoalkyl, each of which has up to 4 carbon atoms and each of which is straight-chain or branched, halogenoalkyl having up to 9 identical or different halogen atoms, and $R^3$, $R^4$, $R^5$, $R^6$ and $R^7$ independently of one another represent hydrogen, fluorine, chlorine, bromine, jodine or nitro, or alkyl, alkoxy, alkylsulphonyl or alkoxycarbonyl, each of which has up to 4 carbon atoms in the particular alkyl parts and each of which is straight-chain or branched, or represent a radical $-(X')_n-R^9$, wherein

X' represents oxygen, sulphur, sulphinyl or sulphonyl, n represents 0 or 1 and

$R^9$ represents straight-chain or branched halogenoalkyl with up to 4 carbon atoms and up to 9 identical or different halogen atoms, with the proviso that at least one of the radicals $R^3$, $R^4$, $R^5$, $R^6$ or $R^7$ represents a radical $-(X')_n-R^9$, but, at the same time, R does not represent cyano and $R^5$ does not represent trifluoromethyl, characterized in that

(a) 5-amino-pyrazoles of the formula (II)

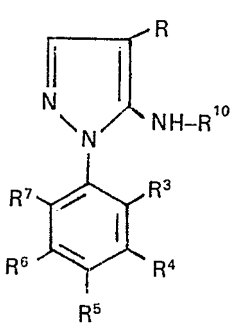

(II)

in which

R, $R^3$, $R^4$, $R^5$, $R^6$ and $R^7$ have the abovementioned meaning and

$R^{10}$ represents hydrogen or a radical $-\underset{\underset{X}{\|}}{C}-R^8$

wherein

X and $R^8$ have the abovementioned meaning, are reacted with alkylating agents of the formula (III)

$R^1$–A (III)

in which

$R^1$ has the abovementioned meaning and

A represents an electron-withdrawing leaving group, if appropriate in the presence of a diluent and if appropriate in the presence of an acid-binding agent, and if appropriate in the presence of a catalyst, or in that

(b) 5-acylaminopyrazoles of the formula (Ia)

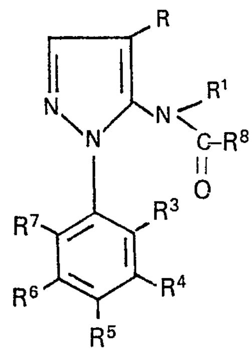

(Ia)

in which

R, $R^1$, $R^3$, $R^4$, $R^5$, $R^6$, $R^7$ and $R^8$ have the abovementioned meaning, are deacylated with acids and bases, if appropriate in the presence of a diluent, or in that

(c) azomethines of the formula (IV)

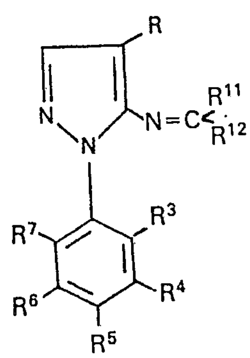

(IV)

in which

R, $R^3$, $R^4$, $R^5$, $R^6$ and $R^7$ have the abovementioned meaning,

$R^{11}$ represents alkyl, alkenyl or alkinyl, each of which has up to 7 carbon atoms and each of which is straight-chain or branched and optionally monosubstituted or polysubstituted by identical or different substituents, possible substituents being: halogen, cyano, hydroxyl and carboxyl, and alkoxy or alkoxycarbonyl, each of which has up to 6 carbon atoms and is straight-chain or branched, and

$R^{12}$ independently of $R^{11}$ represents the same radicals as $R^{11}$, and also represents hyrogen, are reduced with a complex hydride, if appropriate in the presence of a diluent.

4. Herbicidal agents, characterized in that they contain at least one substituted 5-amino-1-phenylpyrazole of the formula (I) according to Claim 1 and 3.

5. Method of combating weeds, characterized in that substituted 5-amino-1-phenylpyrazoles of the general formula (I) according to Claim 1 and 3 are allowed to act on the weeds or their environment.

6. Use of substituted 5-amino-1-phenylpyrazoles of the general formula (I) according to Claim 1 and 3 for combating undesirable plant growth.

7. Process for the preparation of herbicidal agents, characterized in that substituted 5-amino-1-phenylpyrazoles of the general formula (I) according to Claim 1 and 3 are mixed with extenders and/or surface-active agents.

8. Azomethines of the formula (IV)

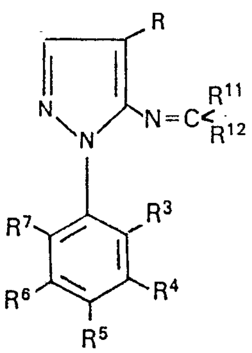

(IV)

in which

R, $R^3$, $R^4$, $R^5$, $R^6$ and $R^7$ have the meaning given in Claim 1,

$R^{11}$ represents alkyl, alkenyl or alkinyl, each of which has up to 7 carbon atoms and each of which is straight-chain or branched and optionally monosubstituted or polysubstituted by identical or different substituents, possible substituents being: halogen, cyano, hydroxyl and carboxyl, and alkoxy or alkoxycarbonyl, each of which has up to 6 carbon atoms and is straight-chain or branched, and

$R^{12}$ independently of $R^{11}$ represents the same radicals as $R^{11}$, and also represents hydrogen.

9. Process for the preparation of azomethines of the formula (IV)

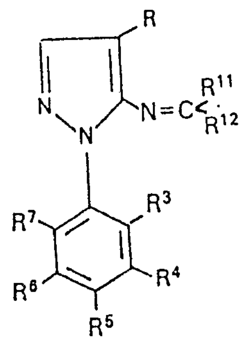

(IV)

R, $R^3$, $R^4$, $R^5$, $R^6$ and $R^7$ have the meanings given in Claim 1,

$R^{11}$ represents alkyl, alkenyl or alkinyl, each of which has up to 7 carbon atoms and each of which is straight-chain or branched and optionally monosubstituted or polysubstituted by identical or different substituents, possible substituents being: halogen, cyano, hydroxyl and carboxyl, and alkoxy or alkoxycarbonyl, each of which has up to 6 carbon atoms and is straight-chain or branched, and

$R^{12}$ independently of $R^{11}$ represents the same radicals as $R^{11}$, and also represents hydrogen, characterized in that 5-aminopyrazoles of the formula (IIa)

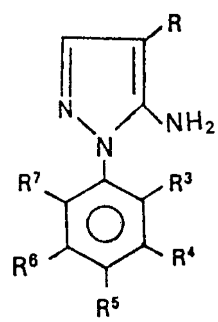

(IIa)

in which

R, $R^3$, $R^4$, $R^5$, $R^6$ and $R^7$ have the meaning given in Claim 1, are reacted with aldehydes or ketones of the formula (XI)

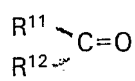

(XI)

in which

$R^{11}$ and $R^{12}$ have the meaning given in Claim 3, if appropriate in the presence of a diluent and if appropriate in the presence of a catalyst at temperatures between +20 °C and +120 °C.